# EUROPEAN PATENT SPECIFICATION

(11) **EP 0 920 354 B1**
(45) Date of publication and mention of the grant of the patent: **03.12.2003**
(21) Application number: 98917560.9
(22) Date of filing: 19.05.1998
(51) Int. Cl.: B01J 31/24, C07C 67/303

(54) **RUTHENIUM CATALYSTS AND THEIR USE IN THE ASYMMETRIC HYDROGENATION OF WEAKLY COORDINATING SUBSTRATES**
RUTHENIUM-KATALYSATOREN UND IHRE VERWENDUNG ZUR ASYMMETRISCHEN HYDRIERUNG VON SUBSTRATEN MIT SCHWACHER KOORDINATION
CATALYSEURS AU RUTHENIUM ET LEUR UTILISATION DANS L'HYDROGENATION ASYMETRIQUE DE SUBSTRATS A FAIBLE COORDINATION

(30) Priority: 20.05.1997 WO PCT/IB97/00575
(43) Date of publication of application: 09.06.1999
(73) Proprietor: FIRMENICH SA, 1211 Genève 8 (CH)
(72) Inventor: DOBBS, Daniel, A., Metuchen, NJ 08840 (US); VANHESSCHE, Koenraad, P., M., Basking Ridge, NJ 07920 (US); RAUTENSTRAUCH, Valentin, CH-1233 Bernex (CH)
(74) Representative: Salvaterra-Garcia, Maria de Lurdes
(86) International application number: IB9800776
(87) International publication number: WO98052687

(56) References cited:
- WO-A-91/02588
- WO-A-96/00206
- WO-A-97/18894
- FR-A- 2 693 190
- US-A- 5 304 524

## Description

### FIELD OF THE INVENTION AND PRIOR ART

The present invention relates to the field of asymmetric hydrogenation and, more particularly, to the use of novel Ru(II) chiral catalysts for the asymmetric hydrogenation of substrates which, as a result of their weak donor capability and of their hindered structures, have proved heretofore very difficult or impossible to hydrogenate.

A great number of chiral metal catalysts has been used in the past to asymmetrically hydrogenate a variety of substrates.

In the context of the present invention, we can cite in particular the efforts of two research groups which actively studied the synthesis of Ru(II) chiral catalysts, obtained from the Ru(II) complex of formula [(COD)Ru(2-methylallyl)₂] (COD=cyclo-1,5-octadiene).

Thus, J.-P. Genet and his collaborators have published work related to catalysts of formula [Ru(P*-P*)(2-methylallyl)₂], wherein P*-P* represents a diphosphine ligand of the type of those currently known under abbreviated designations such as DIOP, CHIRAPHOS, PROPHOS, BDPP, CBD, NORPHOS, DEGUPHOS, BPPM, BINAP, R-DuPHOS (R=methyl or ethyl), BIPHEMP or yet DIPAMP (see, for example, J.-P. Genet et al., Tetrahedron : Asymmetry 1991, 2, 43). Such catalysts were obtained by heating the above-mentioned Ru(II) complex together with the appropriate chelating diphosphine, in a solvent such as hexane or toluene, such as to replace the cyclooctadiene with the chiral phosphine.

Upon subsequent work (see, for example, WO 91/02588 ; J.-P. Genet, Acros Organics Acta, 1994, 1, 1-8 ; J.-P. Genet et al., Tetrahedron : Asymmetry, 1994, 5, 665-690), these authors described the transformation of such catalysts via protonation by means of aqueous acids such as HBr, HCl, HF or HBF₄, in strongly coordinating solvents, capable of playing a role in stabilizing the coordination structure around the metal, which structure, according to the same authors, is of the hexacoordinate type. This kind of catalysts, which can be prepared in situ, proved to be useful for the asymmetric hydrogenation, in protic or strongly electron-donating solvents (methanol, ethanol or their mixtures with other solvents), of substrates comprising carbonyl groups and acyclic ethylenic bonds.

Other studies (see, for example, F. Heiser et al., Tetrahedron : Asymmetry, 1991, 2, 51-62 ; EP 643 052 ; EP 398 132 ; EP 570 674) have resulted in reports of the use of catalysts prepared in situ for hydrogenating a variety of substrates, starting from the same ruthenium complex, but following a process according to which a mixture of said complex and an appropriate diphosphine ligand is treated with namely CF₃COOH, once again in an electron-donor solvent able to stabilize the coordinating structure of the metal.

These catalysts, and others obtained according to similar processes described in the cited references, reveal themselves very efficient in the asymmetric hydrogenation of various substrates, often good electron-donor substrates capable of coordinating the Ru(II), and are typically used with protic solvents, or mixtures of protic and aprotic solvents. However, they proved to be inefficient when used, under the prior art conditions, for the hydrogenation of substrates possessing heavily hindered ethylenic bonds, for example tetrasubstituted double bonds, in particular when the latter are part of ring systems.

In published International patent application N° WO 97/18894, filed on November 20, 1996, we describe new Ru(II) catalysts and teach their successful use for asymmetrically hydrogenating this type of particularly hindered substrates. More particularly, there is described the hydrogenation of cyclopentenone derivatives of formula 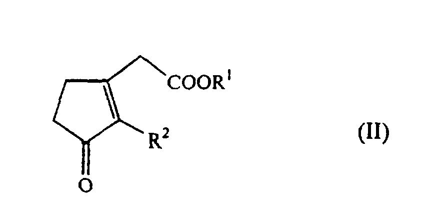 wherein R¹ represents a linear or branched C₁ to C₄ alkyl radical and R² represents a saturated or unsaturated, linear or branched, C₁ to C₈ hydrocarbon radical, the hydrogenation of which had proved impossible until our discovery of novel catalysts obtained by an original process.

The catalysts described in the above-mentioned patent application were obtained by a method which comprised treating equimolar amounts of an appropriate Ru(II) complex, for example [(COD)Ru(2-methallyl)₂] and a chelating diphosphine with an acid of formula HX, wherein X is a non-coordinating anion, said acid being used in a ratio not exceeding 2 molar equivalents per mole of the Ru(II) complex and the treatment being carried out in a non-coordinating or weakly coordinating medium, under an inert atmosphere.

Such catalysts were able to successfully hydrogenate substrates (II), amongst others, to provide their corresponding saturated homologues in strictly cis-configuration and with an enantiomeric excess of at least 60% in the (+)-1R-isomer. Such catalysts, and their use in asymmetric hydrogenation reactions, provided a breakthrough of outstanding importance for the single-step conversion of unsaturated substrates which had previously proved impossible to hydrogenate, into their saturated homologues. Moreover, their use in the conversion of substrates (II) proved particularly valuable in the case of methyl 3-oxo-2-pentyl-1-cyclopentene-1-acetate, the asymmetric hydrogenation of which provided, in a single step, the preferred optically active isomer of methyl dihydrojasmonate or Hedione ® (origin : Firmenich SA, Geneva, Switzerland), a well-known and widely used perfume ingredient.

In fact, amongst the four possible Hedione® stereoisomers, methyl (+)-(1R)-cis-3-oxo-2-pentyl-1-cyclopentaneacetate is known to develop at their best the odor characters, and namely the jasmine note, for which Hedione® is notorious, while the strength of this isomer's odor is also superior to that of the other isomers by several orders of magnitude. Therefore, the production of methyl (+)-(1R)-cis-3-oxo- 2-pentyl-1-cyclopentaneacetate in an optically pure state, or of isomer mixtures which contain essentially this isomer, is of capital importance in the fragrance industry.

### DESCRIPTION OF THE INVENTION

One object of the present invention is a ruthenium catalyst obtainable by a process which comprises putting into contact an appropriate Ru(II) complex, a chelating diphosphine and an acid comprising a non-coordinating anion, said Ru(II) complex and chelating diphosphine being present in equimolar amounts, the contact occurring in a non-coordinating or weakly coordinating medium and under an oxygen-free atmosphere, wherein the acid comprising the non-coordinating anion is used in an amount of about 1 molar equivalent per mole of the Ru(II) complex.

Reference to "about 1 molar equivalent of acid per mole of Ru(II) complex" signifies here a molar ratio between these two components which does not significantly differ from 1, and is preferably comprised within a range of between 0.95 and 1.10, more preferably from 1.0 to 1.10.

The above-mentioned catalysts of the invention provide surprisingly good results when used in the hydrogenation of substrates (II) in particular, but can also be conveniently used for the hydrogenation of substrates of general formula 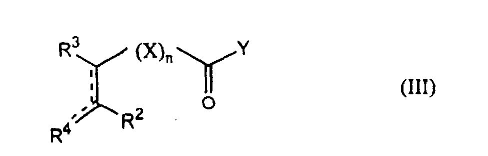 as defined in claim 12. Preferred embodiments of the hydrogenation process of the invention relate to the hydrogenation of substrates (III) having a carbon- carbon double bond in one of the positions indicated by the dotted lines which is at least trisubstituted, i.e. not more than one amongst the R², R³ and R⁴ groups represents hydrogen.

It will be apparent to the person skilled in the art from the disclosure which follows that the catalysts of the invention can also be successfully used in the hydrogenation of ethylenic bonds less sterically hindered than those of preferred substrates (III) described above. However, they are most advantageously used in the one-step conversion of the preferred substrates (III) defined above into their saturated homologues, as is apparent hereafter, particularly those wherein the double bond is tetrasubstituted.

We have in fact ascertained that the catalysts of the invention can be successfully and generally used in asymmetric hydrogenations and are able to readily hydrogenate di-, tri- and tetrasubstituted double bonds, isolated or in α,βor β,γ-position to an ester function, in α,β-position to a ketone or aldehyde function and even in β,γ-position to an alcohol function, the ester and ketone groups being totally unaffected by the hydrogenation.

The catalysts of the invention comprise, or consist essentially of, the reaction product of the appropriate Ru(II) complex, the chelating diphosphine and the acid comprising the non-coordinating anion, the reaction occuring in a non-coordinating or weakly coordinating medium.

By an "appropriate Ru(II) complex" it is meant here any of the Ru(II) complexes currently known and used in the preparation of ruthenium catalysts, amongst which can be cited for example those wherein the metal is surrounded by dienyl and alkyl type ligands, such that the metal is σ-bonded to two of said ligands, which ligands further possess at least one bond π-bonded to the metal, two other coordination positions being π-bonded to the same said two ligands or to a distinct ligand.

Several ruthenium compounds are known from the prior art which comprise ligands fulfilling the above-mentioned conditions and which are convenient as precursors of the catalysts of the present invention.

One can cite more particularly, as appropriate ruthenium(II) complexes, the compounds of the [(diene)Ru(allyl)₂] type, wherein "diene" stands for example for COD (cycloocta-1,5-diene) or NBD (norbomadiene), or yet hepta-1,4-diene, and "allyl" represents a n allyl or 2-methallyl radical (see, for instance, J.-P. Genet et al., cited references ; M.O. Albers et al., Inorganic Synth., 1989, 26, 249 ; R.R. Schrock et al., J. Chem. Soc. Dalton Trans., 1974, 951). Other appropriate ruthenium(II) complexes include the compounds of the [bis(pentadienyl)Ru] type, wherein "pentadienyl" stands for a pentadienyl, 2,4-dimethylpentadienyl, 2,3,4-trimethylpentadienyl, 2,4-di(tert-butyl)pentadienyl or yet 2,4-dimethyl-1-oxapentadienyl radical (see, for example, R.D. Ernst et al., J. Organometallic Chem., 1991, 402, 17 ; L. Stahl et al., Organometallic 1983, 2, 1229 ; T. Schmidt et al., J. Chem. Soc. Chem. Commun., 1991, 1427 ; T.D. Newbound et al., Organometallics, 1990, 9, 2962).

Yet other appropriate Ru(II) complexes include [Ru(COD)(COT)], wherein COT stands for cycloocta-1,3,5-triene, [bis(2,4-cyclooctadienyl)Ru] and [bis(2,4- cycloheptadienyl)Ru] (see, for example, P. Pertici et al., J. Chem. Soc. Dalton Trans., 1980, 1961 ; Inorganic Synthesis 1983, 22, 176) and [Ru(NBD)(CHT)] wherein NBD is norbornadiene and CHT stands for cyclohepta-1,3,5-triene (see for example, H. Nagashima et al., J. Organometallic Chem. 1983, 258, C15).

Following a preferred embodiment of the catalysts of the invention, there is used as the Ru(II) precursor, the compound of formula : [(COD)Ru (2-methallyl)₂], bis(2,4-dimethylpentadienyl)ruthenium (e.g. L. Stahl et al. or T.D. Newbound et al., references cited), bis(2,4-dimethyl-1-oxapentadienyl) ruthenium complexes (e.g. T. Schmidt et al., reference cited) or yet [Ru(COD)(COT)] (P. Pertici et al., ref. cited). [(COD)Ru(2-methallyl)₂], the preparation of which was first reported by J. Powell et al., in J. Chem. Soc., (A), 1968, 159 (see also M.O. Albers et al., Inorganic Synthesis 1989, 26, 249, and [Ru(COD)(COT)] proved quite convenient from a practical point of view.

Amongst the chelating diphosphines which can be used as ligands in the catalysts of the invention, there can be cited as preferred embodiments those selected amongst the known chiral diphosphine or bis(phosphine) ligands which make it possible to obtain catalytic species convenient for homogeneous asymmetric hydrogenations. More particularly, such chiral diphosphines include those known under the abbreviations of Me-DuPHOS, Et-DuPHOS, BINAP. TolBINAP, SKEWPHOS, DIPAMP and CHIRAPHOS, the structures of which are represented hereafter for one of the enantiomers in particular, and wherein Ph stands for a phenyl group :

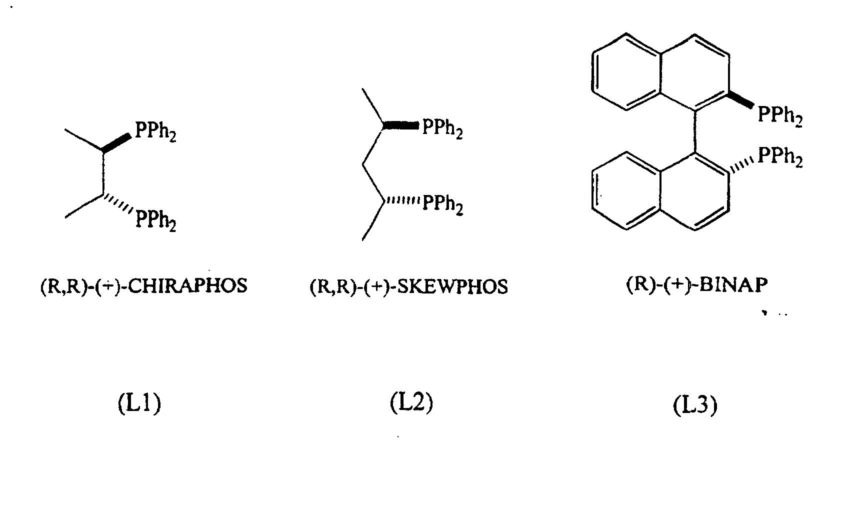

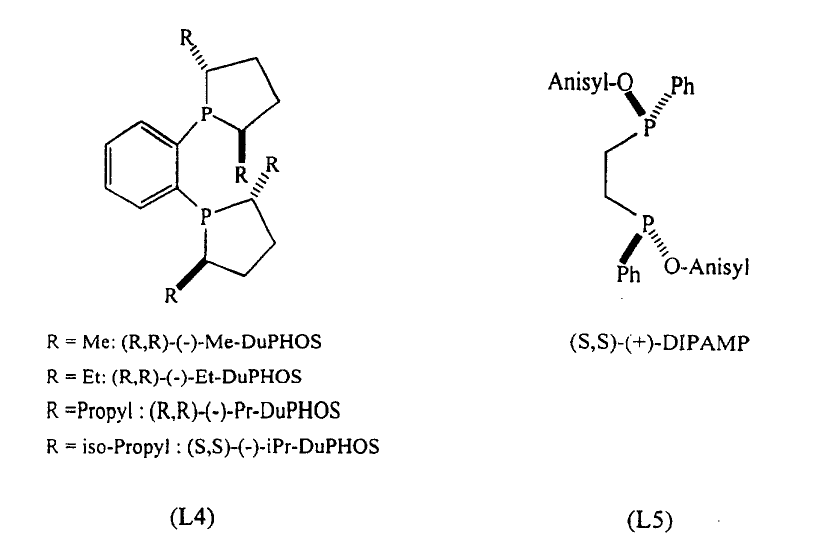

Other chiral bidentate phosphines which can be used in the chiral catalysts of the invention include for instance those known under the name of NORPHOS, or yet the analogues of the DuPHOS type ligands, so-called "BPE", the structures of which are represented hereafter for one of the enantiomers.

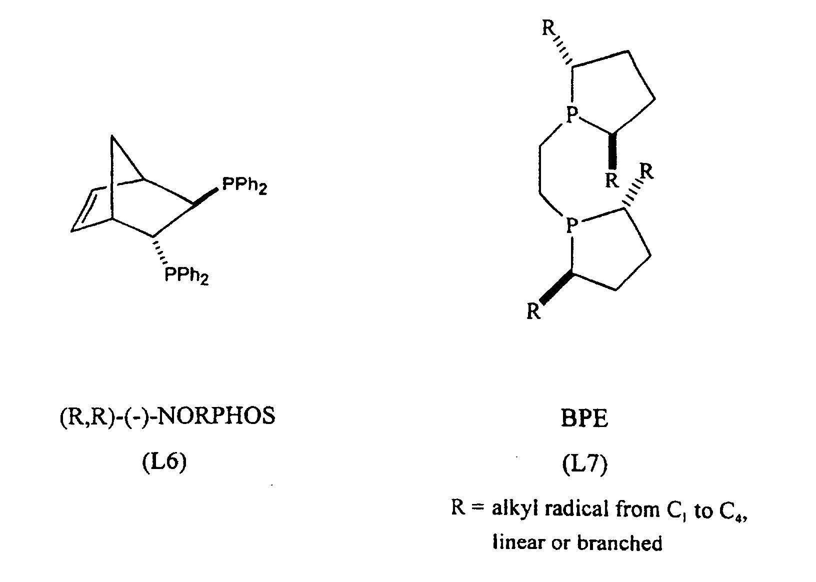

All such ligands are either commercially available or can be prepared by methods previously reported in the literature.

Other particularly useful ligands for the preparation of the catalysts of the invention are the chiral diphosphines described for example in European patent applications nos. 564 406, 612 758 and 646 590, which disclose a large number of ligands appropriate for the catalysts according to the present invention. The contents of these documents, inasmuch as they relate to the definition and preparation or said chelating diphosphines, are hereby included by reference.

Amongst the chelating diphosphines described in EP 564 406, EP 612 758 and 646 590, it is preferred to use those obeying the general formula 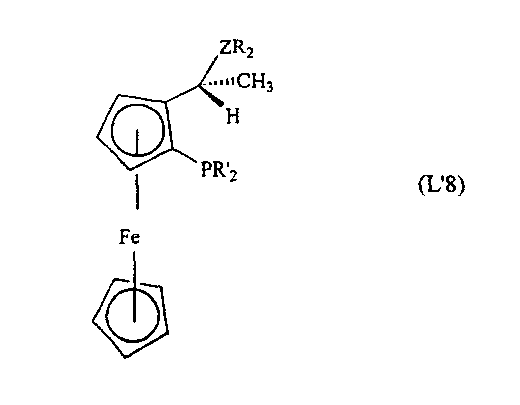 wherein Z is a phosphorus atom and R and R' are, independently from each other, a C₁ to C₄ alkyl group, linear or branched, a cyclohexyl group, a phenyl group or a phenyl group substituted by 1 to 3 alkyl groups having 1 to 4 carbons, the latter alkyl groups possibly being partially or totally fluorinated.

Even more preferred ligands of this type are those of formula 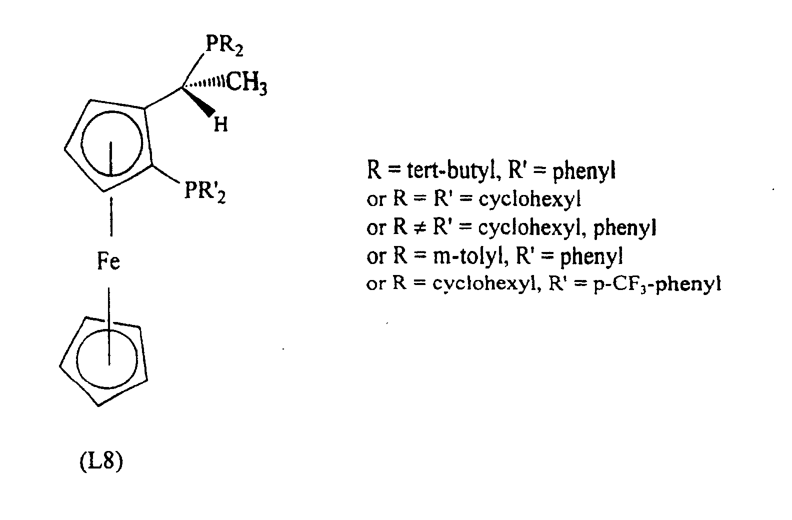 and, more particularly, that known under the designation of (R)-(S)-JOSIPHOS (R=cyclohexyl, R'=phenyl) or (-)-JOSIPHOS, or yet its derivatives such as (R)-(S)-CF₃-JOSIPHOS.

Moreover, we also observed that the catalysts of the invention proved advantageous for the hydrogenation of many substrates, in particular those of formula (II), with a cis-stereoselectivity close to 100%, when they comprised in their structure both chiral and achiral chelating diphosphines. Thus, useful achiral ligands include for example the achiral or racemic ferrocenyl diphosphines represented hereafter:

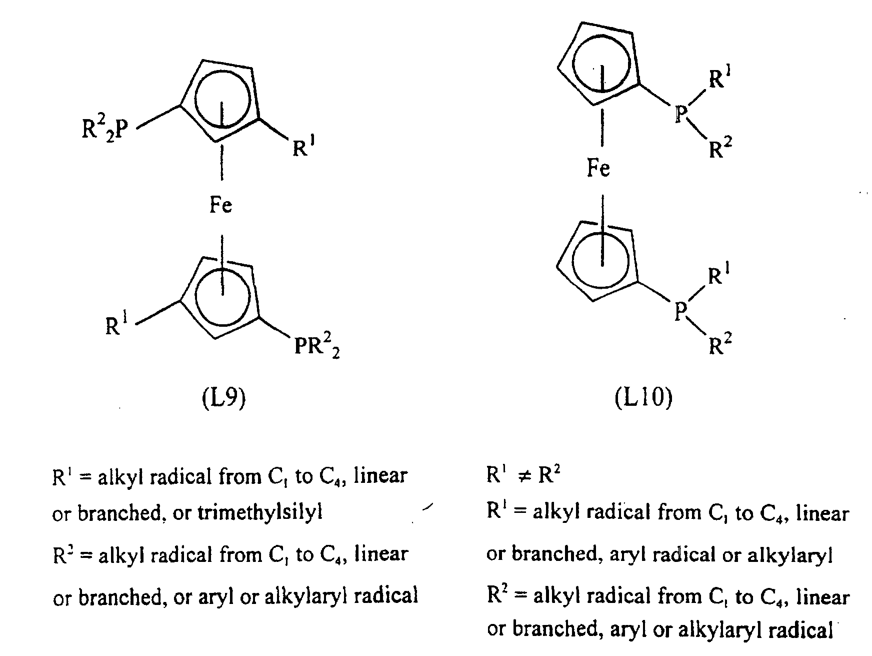

Other bidentate phosphines useful as ligands are represented below :

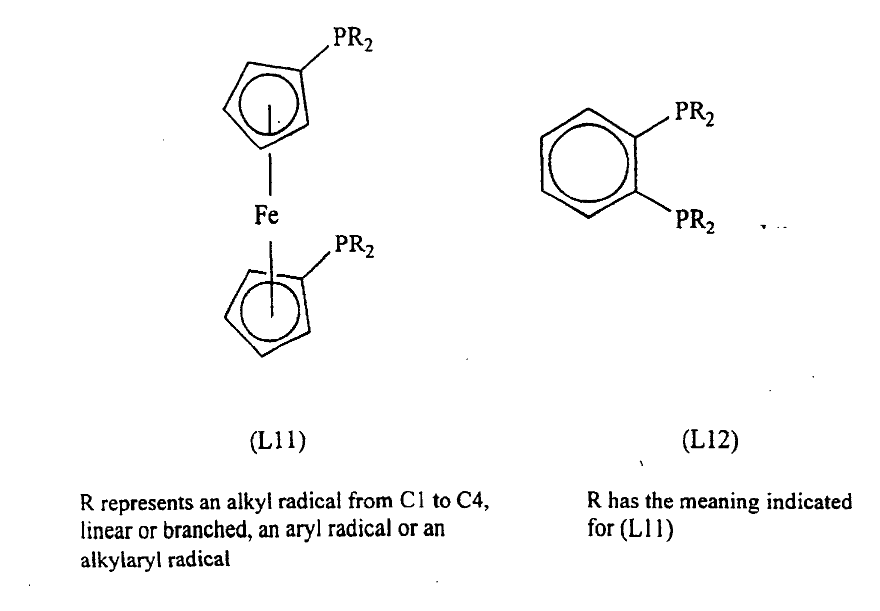

R₂P - (CH₂)ₙ - PR₂

n = 0 to 4
R = cyclopentyl, cyclohexyl or phenyl (L13)

In the context of the invention, there can yet be cited the optically active diphosphines of formula 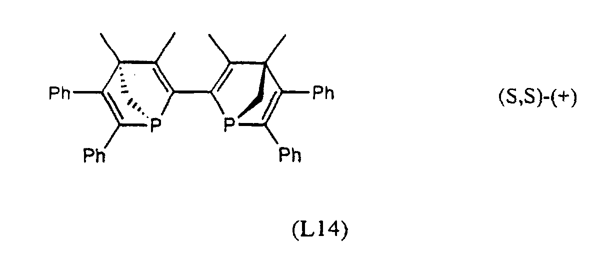 as well as the corresponding racemic mixtures of isomers, reported in International patent application WO 96/20202. The contents of the latter related to the preparation of such ligands are hereby included by reference.

In a general manner, there can be used as ligands in the catalysts of the invention any chelating diphosphines comprising substituent groups capable of rendering the diphosphine sufficiently electron-rich to allow it to stabilize the metal, without however depriving said metal of its ability to coordinate the substrate to be hydrogenated and more particularly substrates (II) and (III) mentioned above, the asymmetric hydrogenation of which had proved heretofore notoriously difficult, if not impossible.

It has also been observed that the diphosphines having a certain number of alkyl or cycloalkyl type substituents revealed themselves particularly useful for the aim of the invention and provided catalysts which were very active and efficient for the hydrogenation of substrates (II) and (III).

It is apparent from the above that the preferred ligands for preparing the catalysts of the invention are diphosphine ligands in which the two phosphorous atoms are bridged by groups of the alkyl, 1,2-benzenyl, bis(naphtalenyl) or yet 1,1'-ferrocenyl type, optionally substituted, said phosphorous atoms further carrying two other substituents, which can be identical or different and formed of alkyl, aryl or alkylaryl radicals, or yet alicyclic radicals.

However, it is impossible to exhaustively illustrate here all the ligands which can be used in the catalysts of the invention and many other chelating diphosphines, not specifically cited above, or not falling under the above definitions, prove useful for the aim of the latter. The examples given illustrate preferred embodiments which are not to be interpreted as restricting the scope of the invention, inasmuch as the person skilled in the art is well able, without particular effort and using her general knowledge to select such ligands so as to achieve the aim described. To this effect, the skilled person can also find inspiration in the many prior art references, namely those cited in this description, to select many such ligands which, when used according to the invention here-described, make it possible to obtain catalysts able to achieve the same effect, i.e. hydrogenate substrates (II) and (III) with essentially cis-stereoselectivity (90% or more) for (II), and providing, where applicable, an excess of at least 60% in one of the cis-enantiomers.

The catalysts of the invention which comprise diphosphine ligands of the DuPHOS, BINAP, TolBINAP, SKEWPHOS or JOSIPHOS type are particularly advantageous as catalysts for the asymmetric hydrogenation of the substrates of formula (II) and (III).

Amongst the latter catalysts, those which comprise ligands of the SKEWPHOS, JOSIPHOS or Me-DuPHOS type, and preferably of the two latter types, showed themselves capable of particularly advantageous performances and are therefore preferred according to the invention. (R,R)-(-)-Me-DuPHOS, or (-)-1,2-bis(2,5-dimethyl-phospholano)benzene, and (R)-(S)-JOSIPHOS or (R)-(S)-CF₃-JOSIPHOS, made it possible to obtain choice catalysts according to the invention.

The ligands of the type L1 to L14 represented below are either commercially available compounds or they can be prepared according to processes analogous to previously described methods.

For example, the ligands of the DuPHOS, SKEWPHOS, BINAP, CHIRAPHOS, DIPAMP and NORPHOS type are mostly commercial products and, in any event, they can be obtained via processes described in the literature, namely in reference works such as the books of R. Noyori, Asymmetric Catalysis in Organic Synthesis, John Wiley & Sons, N.Y. (1994), Chap. II and J. Ojima, Catalytic Asymmetric Synthesis, VCH Publishers, N.Y. (1994). Chap. I. and the original references there-cited wherein such ligands were first reported.

The ligands of type (L7) can be prepared as described for example in US 5,171,892.

The ligands of type (L8) and (L'8) can be prepared as described in the references already cited (see also A. Togni et al., J. Amer. Chem. Soc. 1994, 116, 4062) and some of them are commercially available (R=cyclohexyl, R'=phenyl for example ; origin : STREM Chemicals, Inc.).

The ferrocenyl ligands of type (L9), (L10) or (L11), when not available commercially, can be prepared by methods analogous to those reported in the literature (see, for example, I.R. Butter et al., Synth. React. Inorg. Met. - Org. Chem., 1985, 15, 109 ; M.D. Rausch et al., J. Organometallic Chem., 1967, 10, 127 ; R.A. Brown et al., Polyhedron 1992, 20, 2611 ; G. Herberich et al., Chem. Ber., 1995, 128, 689) starting from ferrocene and according to the following reaction schemes:

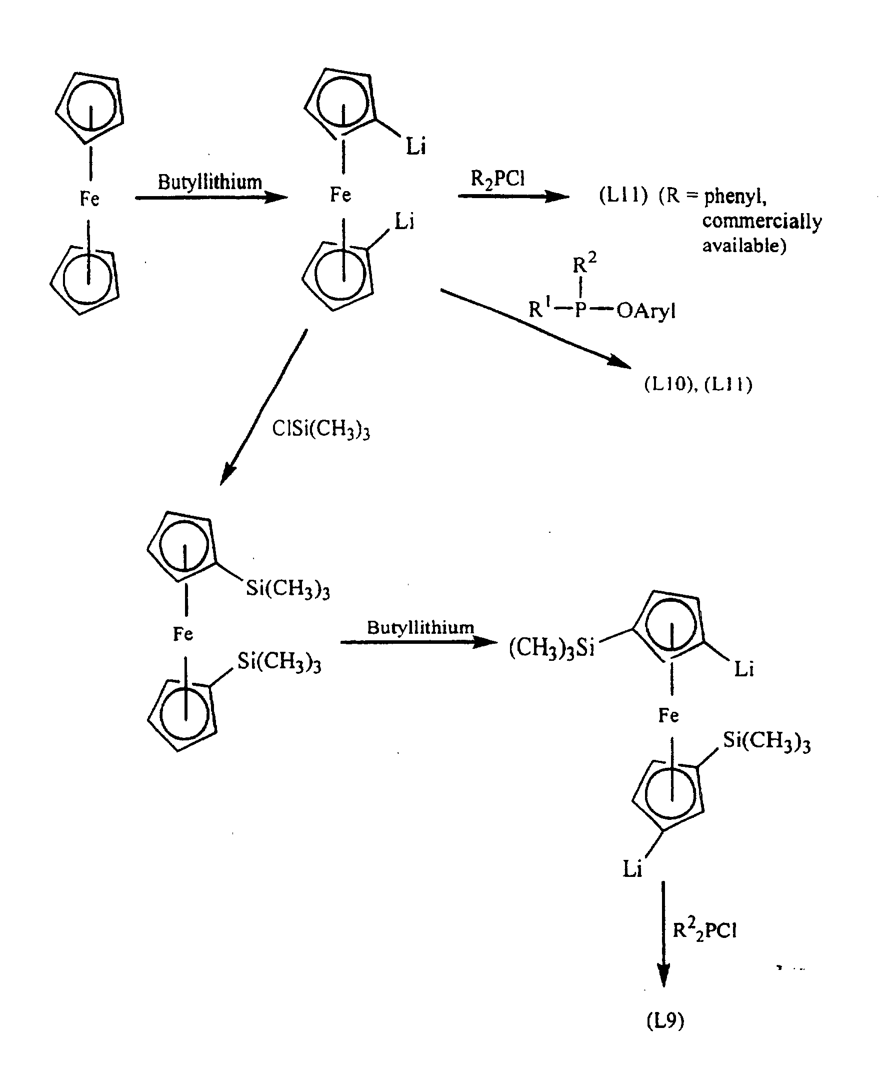

The (L12) and (L 13) ligands are quite common and many are commercialized. Amongst the (L13) type ones, those wherein n=0 can be prepared by a variety of known methods (see, for example, R. Appel et al., Chem. Ber., 1975, 108, 1783 and references cited therein ; M. Baudler et al., Chem. Ber. 1972, 105, 3844 ; K. Issleib et al., Journal für Praktische Chemie, 1969, 311, 463).

The non-coordinating anion to be used in the catalysts of the invention can be derived from a variety of strong acids and includes for example the anions selected from the group consisting of BF₄⁻, PF₆⁻, SbF₆⁻, AsF₆⁻ and B[3,5-(CF₃)₂C₆H₄]₄⁻. Thus, suitable acids from which this anion can be derived include, amongst others, those of formula RBF₄, RPF₆, RSbF₆, RAsF₆, and RB[3,5-(CF₃)₂C₆H₄]₄ wherein R represents hydrogen or a (C₆H₅)₃C group.

According to a preferred embodiment of the invention, the non-coordinating anion is derived from an acid of formula HX, wherein X is selected amongst the anions BF₄⁻, PF₆⁻, SbF₆⁻, AsF₆⁻, or yet HB[3,5-(CF₃)₂C₆H₄]₄⁻. Such acids are typically used in the form of the corresponding etherates (for example HBF₄.R₂O, R=CH₃ or C₂H₅) or of any other onium type salt (phosphonium for example). These etherates are commercial products, or they can be prepared from the corresponding silver salts, by reacting with HCl. In the latter case, the silver salt, for example AgBF₄, AgPF₆, AgSbF₆ or AgAsF_{b} will be typically reacted with HCl, in a solvent containing a dialkylether, for example a mixture of dichloromethane and diethylether. As the silver chloride precipitates, it provides the etherate solution of the acid, which can then be used according to the invention in the reaction with the ruthenium complex and the phosphine ligand.

Amongst the above-mentioned HX acids, wherein X represents the non-coordinating anion, tetrafluoroboric acid is preferred and typically used in the form of its etherate, as commercially available in glass or plastic vials. If necessary, this product is titrated to ensure that the concentration of acid is about 1 molar equivalent of the molar amount of Ru complex.

The reaction according to which the catalyst of the invention can be obtained occurs in a non-coordinating or weakly coordinating medium and under an oxygen-free atmosphere. By an "oxygen-free atmosphere" it is meant here an atmosphere whose oxygen content is lower than 200 ppm, and preferably not above 5 to 10 ppm.

By the "non-coordinating or weakly coordinating medium" it is typically meant here a non-coordinating or weakly coordinating solvent. Examples of suitable solvents include esters, ketones, aliphatic, acyclic or cyclic hydrocarbons, chlorinated hydrocarbons and ethers, as long as they have no capability to strongly coordinate the ruthenium. Specific examples include dichloromethane, dichloroethane, ethyl pivalate, methyl acetate, ethyl acetate, isopropyl acetate, acetone, 2-butanone, 3-pentanone, hexane, heptane, cyclohexane, cycloheptane and methyl tert-butyl ether.

According to a preferred embodiment, dichloromethane or a mixture of it with other, non-coordinating or weakly coordinating solvents, in particular those mentioned above, is used.

It should be noted that the use of the strongly coordinating or protic solvents current in the art for the preparation of Ru catalysts, provided species which were inactive or inadequate for the hydrogenation of the substrates of formula (II) or (III) previously mentioned.

The non-coordinating or weakly coordinating medium may also consist of a mixture of a solvent such as described above with a substrate of formula (II) or (III), or even essentially of just said substrate (II) or (III).

Thus, according to an embodiment of the invention there is provided a catalyst obtainable in the presence of an organic non-coordinating or weakly coordinating solvent and/or of a substrate of formula 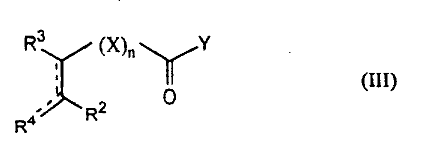 having a double bond in one of the positions indicated by the dotted lines and wherein
a) n=0 and Y represents hydrogen, a C₁ to C₄ linear or branched alkyl radical or an OR¹ group, R¹ standing for a linear or branched lower alkyl radical ;
   or
b) n=1, X represents a CH₂ group and Y stands for an OR¹ group wherein R¹ has the meaning cited in a) ;
   or
c) n=1, X represents an oxygen atom and Y represents a C₁ to C₄ linear or branched alkyl radical, or X represents a NR⁵ group, R⁵ standing for a lower alkyl radical, and Y stands for a linear or branched C₁ to C₄ alkyl radical ;
and wherein
R² represents hydrogen or a saturated or unsaturated, linear or branched C₁ to C₈ radical derived from a hydrocarbon ;
R³ and R⁴ are taken separately and each represents hydrogen or a saturated or unsaturated, linear or branched C₁ to C₈ radical derived from a hydrocarbon, or are taken together to form a five-membered or six-membered ring which also contains the carbon atoms of the ethylenic bond.

Preferred substrates of the invention are compounds of formula (III) wherein not more than one of the R², R³ and R⁴ symbols represents hydrogen.

By a lower alkyl radical it is meant here a C₁ to C₄, linear or branched alkyl radical.

When the catalyst of the invention is obtained in the presence of the substrate, preferred embodiments of said catalyst include the presence of substrates formed of compounds of formula 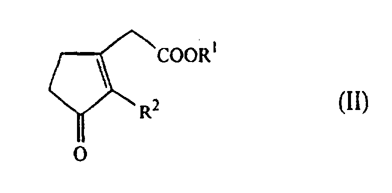 wherein R¹ is a linear or branched alkyl radical from C₁ to C₄ and R² is a saturated or unsaturated, linear or branched, C₁ to C₈ hydrocarbon rest, or of formula 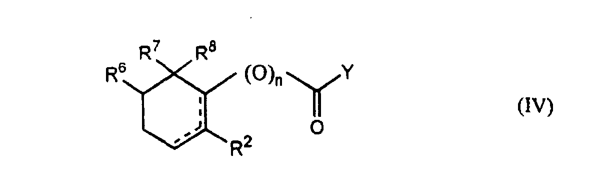 having a double bond in one of the positions indicated by the dotted lines and wherein :
a) n=0 and Y represents an OR¹ group, R¹ standing for a C₁ to C₄ linear or branched alkyl radical ;
   or
b) n=1, and Y represents a C₁ to C₄ linear or branched alkyl radical ;
and wherein R² is a saturated or unsaturated, linear or branched, C₁ to C₈ hydrocarbon rest and R⁶, R⁷ and R⁸ represent each hydrogen or a lower alkyl radical of C₁ to C₄.

In this context, particularly useful catalysts are those wherein the substrate present in the reaction medium is a compound of formula (IV) in which n=0 and Y represents a OR¹ group wherein R¹ represents a methyl or ethyl group, R² represents a methyl radical and R⁶, R⁷ and R⁸ are identical or different and represent each hydrogen or a methyl group.

More preferred substrates are methyl 3-oxo-2-pentyl-1-cyclopentene-1-acetate and methyl 2,6,6-trimethyl-2-cyclohexene-1-carboxylate, which, as will become apparent shortly, make it possible to obtain preferred isomers of useful perfume ingredients, amongst which Hedione®.

As previously mentioned, the medium in which the reaction takes place can also be a mixture of one of the substrates mentioned above and a non-coordinating or weakly coordinating solvent, in which case the latter will be preferably chosen amongst the solvents already cited above, and more preferably as a solvent comprising dichloromethane.

The catalysts of the invention described above are extremely active species which have been found to improve upon those described in the WO 97/18894 patent application. In fact, although the catalysts described in the above document are quite appropriate for the hydrogenation of the same type of ethylenic bonds and substrates as are here contemplated, we have now unexpectedly discovered that complete conversion of these substrates can now be obtained, by means of the presently claimed catalysts, in even shorter reaction times, while using lower amounts of catalyst.

Amongst the catalysts of the invention described above, particularly effective species for the asymmetric hydrogenation of methyl 3-oxo-2-pentyl-1-cyclopentene-1-acetate were those wherein the Ru(II) complex is [(COD)Ru(2-methallyl)₂] and the anion BF₄⁻ derived from HBF₄.etherate, and wherein (R,R)-(-)-Me-DuPHOS or (R)-(S)-JOSIPHOS was used in a medium comprising dichloromethane, in both cases preferably in the presence of the substrate mentioned here-above.

The invention further realizes a process for the preparation of a ruthenium catalyst, characterized in that an appropriate Ru(II) complex and a chelating diphosphine present in equimolar amounts, and an acid comprising a non-coordinating anion, are reacted under an oxygen-free atmosphere and in a non-coordinating or weakly coordinating medium, wherein the acid comprising the non-coordinating anion is used in an amount of about 1 molar equivalent per mole of the Ru(II) complex.

The nature of the parameters of this process, i.e. the Ru(II) complex, the chelating diphosphine, the non-coordinating anion and the reaction medium, have been described in detail above.

According to the invention, the preparation of the catalyst can be carried out at room temperature or at a lower temperature. Higher temperatures may also be used, and can be chosen such as not to influence the catalyst's properties and its efficiency in the hydrogenation of the substrates. Nevertheless, the application of room temperature proves to be advantageous from a practical point of view.

As mentioned before, the catalyst is prepared under inert, oxygen-free atmosphere, typically under argon or nitrogen.

Preferred embodiments of this process correspond to the use of the preferred parameters already described above with regard to the catalysts, e.g. the use of optically active diphosphines, preferred anions and so on.

In particular, the preparation of the catalyst in a medium consisting of a non-coordinating or weakly coordinating solvent and a small amount of substrate, in particular substrates (II) or (IV), turns out to be a very advantageous embodiment of the process of the invention.

The catalysts according to the present invention which have been prepared like this are obtained as solutions, in the solvent and the substrate, of the product which is the result of the reaction of the Ru(II) complex with the diphosphine ligand and the acid from which the anion is derived. These catalytic solutions may be used as such for the asymmetric hydrogenation of the substrates, namely compounds (II) and (IV). They can be kept under an oxygen-free atmosphere and will stay active for several days.

Catalytic solutions according to the present invention are thus obtained, having a variable concentration in the catalyst of the invention, for example of the order of 0.04 to 0.07 M (0.04 to 0.07 mmoles of catalyst/ml of catalytic solution), and more preferably of the order of 0.055 M, which proved to be very advantageous for the hydrogenation of substrates (II) and (IV) in particular.

Preferred pre-catalysts of the invention are those of formula

[Ru (P*-P*) (H) (triene)]⁺ X⁻ (V)

wherein P*-P* represents a chelating bis(phosphine)ligand, possibly chiral, X represents a non-coordinating anion and "triene" stands for cyclohepta-1,3,5-triene, cycloocta-1,3,5-triene or a similar species. These most preferred compounds require the presence of at least one COD molecule in the precursor ruthenium (II) complex. According to a preferred embodiment of this catalyst, the diphosphine is (R,R)-(-)-Me-DuPHOS or (R)-(S)-JOSIPHOS and the anion is BF₄⁻.

Other embodiments of this catalyst are apparent from the text above, the diphosphine ligand and the anion being varied at will, having the meaning previously defined and being specifically cited above with regard to particular examples of the catalysts of the invention.

The preparation of preferred embodiments of the catalysts of formula (V) are described in detail further on.

The ruthenium catalysts of the invention are useful for the hydrogenation, optionally asymmetric, of carbon-carbon double bonds in general and more particularly highly sterically hindered ones.

Their activity with regard to the hydrogenation of the substrates of formulae (II), (III) and (IV) in particular is excellent, unlike what is the case of the prior art ruthenium catalysts. They enable the preparation of the saturated compounds corresponding to substrates (II) and (IV) with a cis-stereoselectivity above 95% and, in most cases of the order of 98% or more. When chiral chelating bis(phosphines) are present in the structure of the catalysts of the invention, the asymmetric hydrogenation of the above-mentioned substrates provides enantiomers with at least 60% e.e., and in many cases above 85% ee.

According to preferred embodiments of the invention, there are thus also provided processes for the preparation of compounds resulting from the hydrogenation of compounds of formula (II) and (IV).

Therefore, the invention also relates to a process for the preparation of a compound of formula 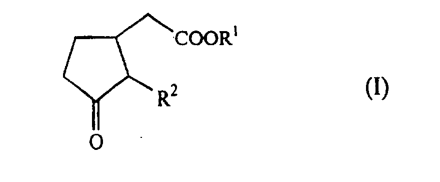 wherein R¹ is a linear of branched C₁ to C₄ alkyl radical and R² is a saturated or unsaturated, linear or branched C₁ to C₈ hydrocarbon rest, essentially in the form of an isomer of cis-configuration, characterized in that a substrate of formula 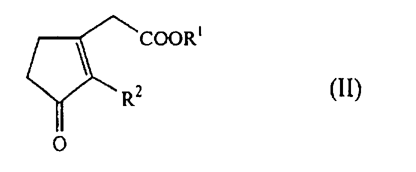 in which R¹ and R² have the meaning indicated above, is hydrogenated in the presence of a Ru catalyst as previously described and at a hydrogen pressure comprised between atmospheric pressure and 500 bar (5x10⁷ Pa).

Another object of the invention is a process for the preparation of a compound of formula 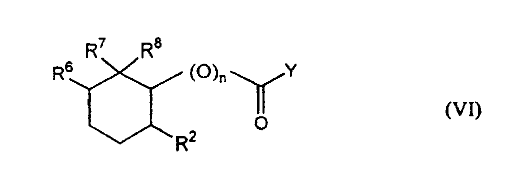 wherein the dotted lines and the symbols n, Y, R², R⁶, R⁷ and R⁸ are defined as in formula (IV), essentially in the form of an isomer of cis-configuration, characterized in that a substrate of formula (IV) as defined above is hydrdgenated in the presence of a ruthenium catalyst according to the invention and at a hydrogen pressure comprised between atmospheric pressure and 500 bar.

Preferred embodiments of these two processes resort to the use of catalysts which comprise appropriate chiral chelating diphosphines, such as to provide the compound of formula (I) or (VI) essentially in the form of an optically active isomer of cis-configuration.

As is current in the art, the substrate is preferably used in a pure state and free of oxygen.

The hydrogenation reaction can be carried out in a non-coordinating or weakly coordinating solvent, the latter being defined as already described above. Specific examples thus include dichloromethane, dichloroethane, ethyl pivalate, methyl acetate, ethyl acetate, isopropyl acetate, acetone, 2-butanone, 3-pentanone, hexane, heptane, cyclohexane, cycloheptane and methyl tert-butyl ether, and their mixtures. The use of a solvent comprising dichloromethane is preferred.

Alternatively, the hydrogenation medium in which the reaction takes place may consist essentially of the substrate, or be highly concentrated in the latter.

According to a preferred embodiment of the hydrogenation process of the invention, the catalyst is generated in situ before the hydrogenation of the substrate, or in the presence of a small amount of the latter.

All the racemic or achiral ligands previously cited can be used to generate catalysts which make it possible to prepare the compounds of formula (I) or (VI) essentially in the form of the cis-configuration isomer, whereas the optically active chelating diphosphines provide the desired optically active cis form of the cited compounds (I) or (VI).

Of course, racemic ligands can be subjected to separation, for example by means of chiral columns, to provide the corresponding enantiomers.

Following a particularly preferred embodiment of the hydrogenation process according to the present invention, methyl 3-oxo-2-pentyl-1-cyclopentene-1-acetate is used as the substrate, in order to obtain Hedione® in the form of the preferred cis- and (+)-cis-configuration isomers.

The catalysts according to the present invention which start from the [(COD)Ru(2-methallyl)₂] precursor and have ligands of the DuPHOS type, and in particular Me-DuPHOS, have proved to be particularly useful for the hydrogenation of this substrate, carried out in a solvent of dichloromethane.

Other preferred conditions for the hydrogenation of this substrate include the use of a catalyst comprising [(COD)Ru(2-methallyl)₂] and (R)-(S)-JOSIPHOS as the ligand, in a hydrogenation medium comprising a hydrocarbon such as hexane, heptane or their saturated cyclic analogues, and more preferably methyl tert-butyl ether.

This latter preferred embodiment of the hydrogenation process of the invention makes it possible to obtain (+)-cis-Hedione® with an enantiomeric excess of 80% or more. Still another preferred embodiment resorts to the use of [Ru(COD)(COT)] and (R)-(S)-JOSIPHOS.

The hydrogenation can be carried out at pressures from about atmospheric pressure to 500 bar (5x10⁷ Pa). Pressure values comprised between 20, and more preferably 50 to 200 bar or even higher are quite convenient.

The reaction may take place at temperatures of up to 50 to 60°C, or even 100°C. Preferably, one will work at room temperature or even a lower temperature. It has been found for example that temperatures down to -10°C, or even lower, made it possible to obtain good results.

The molar concentrations in which these catalysts may be typically used can go up to 4 molar %, relative to the substrate, but preferred embodiments of the hydrogenation process of the invention comprise using the catalyst in a molar concentration of about 0.01 to 1%, more preferably between 0.01 and 0.5 molar %. Excellent results could be systematically obtained with concentrations of 0.02 to 0.1%, more particularly 0.025 to 0.05 molar %, relative to the substrate.

Moreover, it has also been found that it is advisable to use solutions in which the substrate is concentrated, and the best results have been obtained when the concentration of the substrate in the hydrogenation medium was from about 0.4 to 1.5 molar with respect to the volume of this medium.

To hydrogenate the reaction mixture, the latter is pressurized under hydrogen in a conventional manner and as is described in the examples below. With the catalysts of the invention, used in concentrations of the order of 0.05 molar % relative to the substrate complete conversion of the latter could be obtained in less than 24 h, and in the best cases in 6 hours or less.

The invention will now be described in greater detail by way of the following examples, wherein the temperatures are indicated in degrees Celsius and the abbreviations have the usual meaning in the art.

These examples illustrate particular and best embodiments of the invention. many variations of which can be readily construed from the description above. Namely. in the many examples of catalysts taught in the WO 97/18894 application, which resulted from the various combinations of the preparation parameters also described in detail in the preceding pages of the instant disclosure, the use of about 1 molar equivalent of acid comprising a non-coordinating anion per mole of Ru(II) complex, as instantly taught, provided enhanced novel catalysts. The contents of this WO 97/18894 application is therefore hereby included by reference, to the extent that it provides specific examples of the many catalysts that can be obtained by varying the nature of the Ru(II) complex precursor. of the diphosphine ligand, of the non-coordinating anions and their precursors. and of the non-coordinating or weakly-coordinating solvent.

It is to be appreciated that the use of the respective enantiomers of the ligands mentioned throughout this specification, for example of (S,S)-(+)-MeDuPHOS or (S)-(R)-JOSIPHOS, makes it possible to obtain the corresponding enantiomers of compounds (I) and (VI) mentioned above.

In the examples hereafter, reference to a "glovebox" means a glovebox under Ar or nitrogen and the oxygen content of which is under 10 ppm.

### Example 1

### Preparation of [Ru((R,R)-Me-DuPHOS)(H)(COT)] (BF₄)

In a glovebox, there were charged into a 50 ml flask 260.7 mg (0.816 mmole) of [(COD)Ru(methallyl)₂] (origin : Acros Organics) and 10.8 ml of methylacetate. A separate 10 ml vial was charged with 250 mg (0.816 mmole) of (R,R)-(-)-Me-DuPHOS (origin : STREM Chemicals), 5 ml of CH₂Cl₂ and 137 µl of 81% HBF₄.etherate (0.816 mmole of HBF₄). The contents of this 10 ml vial were then poured into the stirred mixture contained in the 50 ml flask and this mixture was further stirred during 12 hours. A yellow precipitate was collected by filtration (180 mg, 0.299 mmole, yield : 37%), consisting of [Ru((R,R)-Me-DuPHOS) (H)(COT)]⁺BF₄⁻ which was extensively analyzed by spectroscopy. The structure of this crystalline product was unambiguously established by spectroscopic analysis. The data obtained were the following (¹H and ¹³C NMR established relative to trimethylsilane ; Cl = Chemical Ionisation) :
¹H NMR(CD₂Cl₂) δ 7.58(m, 4H) ; 6.57(t, J=8.4Hz, 1H) ; 6.30(dd, J₁=6.4Hz, J₂=8.9Hz, 1H) ; 5.62(m, 2H) ; 5.37(q, J=8.4Hz, 1H) ; 5.26(t, J=7.9Hz, 1H) ; 2.71(m, 2H) ; 2.55-2.15(m, 5H) ; 1.33(dd, J₁=6.9Hz, J₂=17.2Hz, 3H) ; 1.30(m, 1H) ; 1.13(dd, J,=6.9Hz, J₂=17.2Hz, 3H) ; 0.93(dd, J₁=6.9Hz, J₂=17.2Hz, 3H) ; 0.70(dd, J₁=6.9Hz, J₂=17.2Hz, 3H) ; -9.94(t, J=29Hz, 1H).
¹³C(¹H) NMR (CD₂Cl₂) δ 141.2(m, C), 141.0(m, C), 132.0(d, J_{pc}=14Hz, CH), 131.5(d, J_{pc}=14Hz, CH), 131.3(s, CH), 131.0(s, CH), 102.3(s, CH), 101.2(s, CH), 99.2(s, CH), 96.2(s, CH), 94.3(s, CH), 94.3(s, CH), 44.5(d, J_{pc}=38Hz, CH), 44.4(d, J_{pc}=14Hz, CH), 40.9(d, J_{pc}=32Hz, CH), 40.1(d, J_{pc}=24Hz, CH), 37.5(s, CH₂), 37.4(s, CH₂), 36.4(s, CH₂), 35.9(s, CH₂), 34.8(s, CH₂), 32.0(s, CH₂), 18.3(s, CH₃), 16.2(d, J_{pc}=6.0Hz, CH,), 14.4(s, CH₃), 12.6(s, CH₃) ;
¹⁹F NMR (CD₂Cl₂) δ 10.5 (relative to C₆F₆) ;
³¹P NMR (CD₂Cl₂) δ 87.5 (d, Jₚₚ=20.0Hz), 84.9 (d, Jₚₚ=20.0Hz) (relative to 85% aqueous H₃PO₄) ;
MS (CI) 515, 339, 287, 231, 209, 193, 180.

The identity of this compound was also ascertained by mass spectrometry, ¹H, ¹³C, ¹⁹F, ³¹P NMR spectroscopy, DEPT and ¹H - ¹H, ¹H - ¹³C correlations. The mass spectrum gives a molecular ion for the cationic portion of this complex at 515 m/e. The ¹⁹F NMR spectrum displays a single resonance for a BF₄⁻ moiety indicating the existence of one species. The ³¹P NMR spectrum displays two doublets consistent with one species containing one DuPHOS ligand with inequivalent phosphorous atoms. The ¹H NMR spectrum clearly indicates the presence of a hydride bound to ruthenium, coupled to two phosphorous atoms at -9.94 ppm. Further, there are six resonances for the CH protons on the cyclooctatriene ligand (which was also supported by a ¹H - ¹³C correlation). ¹³C (¹H) and DEPT experiments are also consistent with this structure. There are six resonances for the cyclooctatriene ligand between 103 and 94 ppm indicating the alkenyl nature of this ligand. The methylene groups on the COT ligand were assigned and are at 35.9 and 32.0 ppm. Further, all remaining ¹³C resonances are consistent with a single DuPHOS ligand attached to ruthenium.

Finally, the structure of the precatalyst mentioned above was also confirmed by X-ray analysis.

The concentration of the commercial HBF₄.etherate in HBF₄ could be carefully monitored before its use, according to the following method.

In a glovebox, a 10 ml glass vial was charged with 20.0 mg (0.050 mmole) of (Ph)₂PCH₂CH₂P(Ph₂) (origin : STREM Chemicals) and 500 µl of CH₂Cl₂. To this solution there were added 500 µl of an approximately 0.1 M solution of HBF₄.etherate/CH₂Cl₂. The solution was placed in a NMR tube, sealed and placed in a NMR probe at -70°C. A ³¹P- proton coupled spectrum was collected and integrated. By comparing the integral of free (Ph)₂PCH₂CH₂P(PH₂) with the resonances of the protonated diphosphine a true value for the titre of the HBF₄.etherate/CH₂Cl₂ was obtained.

The title compound was also obtained using [Ru(COD)(COT)] as the starting ruthenium complex.

### Example 2

### Hydrogenation of methyl 3-oxo-2-pentyl-1-cyclopentene-1-acetate

In a glovebox, into a 10 ml glass vial were placed 15.5 mg (0.0485 mmole) of [(COD)Ru(methallyl)₂], 14.9 mg (0.0485 mmole) of (R,R)-Me-DuPHOS and 250 µl of methyl 3-oxo-2-pentyl-1-cyclopentene-1-acetate (1.08 mmole). To this suspension was added 0.630 ml of a 0.081 M solution of titrated HBF₄.etherate (0.049 mmole) in CH₂Cl₂ with stirring. This concentrated solution was stirred for 1.5 hours, after which 21.5 g (95.9 mmole) of methyl

3-oxo-2-pentyl-1-cyclopentene-1-acetate and 11.5 ml of CH₂Cl₂ were added. The resulting yellow solution was placed in a 75 ml autoclave, purged with hydrogen and pressurized to 90 bar of hydrogen. After 8 hours, the solution was exposed to air and passed through a column of silica gel to separate the catalyst. This gave a 99% yield of the desired Hedione® product with the following ratios of isomers : cis/trans = 98/2 ; (+)-cis/(-)-cis = 82.4/17.6, 64.8% ee.

As prepared above, the catalyst of the invention was present in the reaction medium in 0.05 molar % relative to methyl 3-oxo-2-pentyl-1-cyclopentene-1-acetate.

A similar procedure, but adding methyl 3-oxo-2-pentyl-1-cyclopentene-1-acetate neat, provided a 99% yield of the desired Hedione® product with the following ratios of isomers : cis/trans = 98/2 ; (+)-cis/(-)-cis = 83.4/16.6, 66.8% ee.

### Example 3

### Hydrogenation of methyl 3-oxo-2-pentyl-1-cyclopentene-1-acetate using the catalyst described in Example 1

In a glovebox, into a 10 ml glass vial were placed 15.0 mg (0.0250 mmole) of [((R,R)-Me-Duphos)Ru(H)(cyclooctatriene)]BF₄ , 5.62 ml of CH₂Cl₂ and 11.23 g (50.0 mmole) of methyl 3-oxo-2-pentyl-1-cyclopentene-1-acetate. The resulting yellow solution was placed in a 75 ml autoclave, purged with hydrogen and pressurized to 90 bar of hydrogen. After 6 hours, the solution was exposed to air and passed through a column of silica gel to separate the catalyst. This gave a 99% yield of the desired Hedione® product with the following ratios of isomers : cis/trans = 98/2 ; (+)-cis/(-)-cis = 82.3/17.7, 64.6% ee.

Using in the above-described process 7.5 mg of catalyst (0.0125 mmole) provided a final product with the same characteristics as above, in 98% yield, after 33 h of reaction.

### COMPARATIVE EXAMPLE 1

### Hydrogenation of methyl 3-oxo-2-pentyl-1-cyclopentene-1-acetate

Proceeding in a similar manner to that described in Example 2, but in the presence of a catalyst prepared according to the process described in WO 97/18894, i.e. using equimolar amounts of [(COD)Ru(2-methallyl)₂] and diphosphine ligand (see table below), HBF₄.etherate in an amount of 2 molar equivalents per mole of [(COD)Ru(2-methallyl)₂], in dichloromethane or a mixture of the latter with other solvents (see table), ethyl 3-oxo-2-pentyl-1-cyclopentene-1-acetate was hydrogenated under a variety of conditions as detailed in the table below, to provide Hedione® in the form of a variety of products as cited in the table. The molar concentration of the substrate in the hydrogenation medium was always approximately the same to avoid dilution effects.

**TABLE I**

| Ligand | Hydrogenation | Acid solvent | Molar % of catalyst relative to substrate | H₂ pressure (10⁶ Pa) | Reaction time h | Substrate conversion % | cis/ trans ratio | (+)-cis/ (-)-cis ratio |
|---|---|---|---|---|---|---|---|---|
| (R,R)-Me-DuPHOS | CH₂Cl₂ | CH₂Cl₂ | 2 | 9 | 0.5 | 99 | 97:3 | 80:20 |
| (R,R)-Me-DuPHOS | CH₂Cl₂ | CH₂Cl₂ | 1 | 9 | 1.5 | 99 | 98:2 | 80:20 |
| (R,R)-Me-DuPHOS | CH₂Cl₂ | CH₂Cl₂ | 0.5 | 9 | 20 | 99 | 97:3 | 80:20 |
| (R,R)-Me-DuPHOS | CH₂Cl₂ | CH₂Cl₂ | 0.3 | 5 | 70 | 99 | 99:1 | 84:16 |
| (R)-(S)-JOSIPHOS | hexane | CH₂Cl₂ | 0.2 | 3.5 | 20 | 99 | 98:2 | 93:7 |
| (R)-(S)-JOSIPHOS | hexane | CH₂Cl₂ | 0.1 | 4 | 18 | 98 | 72:28 | 90:10 |
| | | | | | | | | |
| (R)-(S)-JOSIPHOS | MTBE* | CH₂Cl₂ | 0.1 | 4 | 20 | 98 | 96:4 | 89:11 |
| (R)-(S)-JOSIPHOS | isopropyl ether | CH₂Cl₂ | 0.1 | 4 | 18 | 98 | 97:3 | 89:11 |
| (R)-(S)-JOSIPHOS | cyclohexane | CH₂Cl₂ | 0.2 | 3.5 | 20 | 96 | 98:2 | 93:7 |
| (R)-(S)-JOSIPHOS | heptane | CH₂Cl₂ | 0.1 | 4 | 18 | 97 | 80:20 | 90:10 |

| | | | | | | | | |
|---|---|---|---|---|---|---|---|---|
| * methyl tert-butyl ether | | | | | | | | |

It appears clearly from this table that far longer reaction times are needed with the method described in WO 97/18894 to obtain full conversion of the substrate, when the catalyst containing (R,R)-MeDuPHOS is used in a molar concentration below 0.3%, whereas with the corresponding catalysts of the instant invention the conversion can be complete in 6 h even when the catalyst is used at 0.05 molar % relative to the substrate.

### COMPARATIVE EXAMPLE 2

### Hydrogenation of methyl 2,6,6-trimethyl-1-cyclohexene-1-carboxylate

Proceeding as described in WO 97/18894 and in comparative Example 1, the above substrate was hydrogenated at a pressure close to 100 bar (10⁷ Pa) and at room temperature, using a catalyst which comprised a variety of ligands, as indicated in the table hereafter. In all the trials, 2 molar equivalents of HBF₄.etherate were used per mole of [(COD)Ru(methallyl)₂] and the catalysts thus obtained were employed at 2 molar % concentration with respect to the substrate. The conversion rates, and the isomer characteristics of the methyl (1S,2S)-2,2,6-trimethyl-1-cyclohexane-carboxylate obtained are indicated in Table 2 hereafter :

**TABLE II**

| Ligand | Reaction time h | Substrate conversion % | Cis/trans ratio | Enantiomeric excess in (1S,2S)-isomer % |
|---|---|---|---|---|
| (S)-TolBINAP | 24 | 96 | 99:1 | 84 |
| (R)-TolBINAP | 21 | 96 | 99:1 | 85 |
| (-)-MeDuPHOS | 21 | 98 | 99:1 | 58 |
| (S)-BINAP | 24 | 71 | 99:1 | 85 |

### Example 4

### Hydrogenation of methyl 3-oxo-2-pentyl-1-cyclopentene-1-acetate

To a cooled solution of [Ru(COD)(COT)] (30.1 mg, 0.095 mmole) in CH₂Cl₂ (5.0 ml) there was added dropwise, under stirring, a diethyl ether solution (54% weight) of HBF₄ (12.0 µl, 0.087 mml). (R)-(S)-JOSIPHOS (56.4 mg, 0.095 mmole) in CH₂Cl₂ (5.0 ml) was then added and the solution was allowed to stir for 6 h and then allowed to warm up to room temperature. Methyl 3-oxo-2-pentyl-1-cyclopentene-1-acetate was then added (catalyst relative to substrate : 0.05%) in methyl tert-butyl ether (v/v = 1:1) and the hydrogenation carried out under the usual conditions (90 bar). After 8.5 h there was obtained the desired product with a ratio cis/trans = 99/1 and (+)-cis/(-)cis = 88/12.

## Claims

1. A ruthenium catalyst comprising the reaction product of an appropriate Ru(II) complex, a chelating diphosphine and an acid comprising a non-coordinating anion, said Ru(II) complex and chelating diphosphine being present in equimolar amounts, the reaction occurring in a non-coordinating or weakly coordinating medium and under an atmosphere having an oxygen content lower than 200 ppm, and the acid comprising a non-coordinating anion being used in an amount comprised between 0.95 and 1.10 molar equivalent per mole of the Ru(II) complex.

2. A catalyst according to claim 1, consisting essentially of said reaction product.

3. Catalyst according to claim 1 or 2, wherein the chelating diphosphine is a chiral diphosphine ligand.

4. Catalyst according to claim 3, wherein the Ru(II) complex is selected from the group of Ru(II) compounds of the type [(diene)Ru(allyl)₂] or [bis(pentadienyl)Ru].

5. Catalyst according to claim 3, wherein the ruthenium complex is [(COD)Ru(2-methallyl)₂] or [Ru(COD)(COT)].

6. Catalyst according to any one of claims 2 to 5, wherein the chelating diphosphine is selected from the group consisting of the chiral ligands known under the abbreviations of Me-DuPHOS, Et-DuPHOS, BINAP, TolBINAP, SKEWPHOS and JOSIPHOS.

7. Catalyst according to claim 6, wherein the bidentate phosphine ligand is selected from the group of chiral diphosphines known under the abbreviations of Me-DuPHOS, SKEWPHOS and JOSIPHOS.

8. Catalyst according to claim 7, wherein the ligand is (R,R)-(-)-Me-DuPHOS, (R)-(S)-JOSIPHOS or (R)-(S)-CF₃-JOSIPHOS, and preferably (R,R)-(-)-MeDuPHOS or (R)-(S)-JOSIPHOS.

9. Catalyst according to any one of the preceding claims, wherein the non-coordinating anion is derived from an acid of formula HX, wherein X is selected from the group consisting of BF₄⁻, B[3,5-(CF₃)₂C₆H₄]₄⁻, PF₆⁻, SbF₆⁻ and AsF₆⁻.

10. Catalyst according to claim 9, wherein the non-coordinating anion is BF₄⁻, preferably derived from HBF₄.etherate.

11. Catalyst according to any one of claims 1 to 10, wherein the weakly coordinating medium is a solvent selected from the group consisting of dichloromethane, dichloroethane, ethyl pivalate, methyl acetate, ethyl acetate, isopropyl acetate, acetone, 2-butanone, 3-pentanone, hexane, heptane, cyclohexane, cycloheptane and methyl tert-butyl ether and mixtures thereof.

12. Catalyst according to any one of claims 1 to 11, wherein the non-coordinating or weakly coordinating medium is formed of a non-coordinating or weakly coordinating organic solvent and/or a substrate of formula 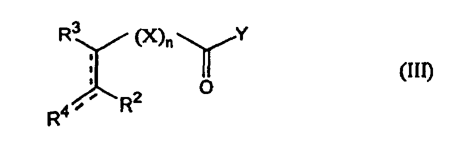 having a double bond in one of the positions indicated by the dotted lines and wherein
a) n=0 and Y represents hydrogen, a C₁ to C₄ linear or branched alkyl radical or an OR¹ group, R¹ standing for a linear or branched lower alkyl radical ;
or
b) n=1, X represents a CH₂ group and Y stands for an OR¹ group wherein R¹ has the meaning cited in a) ;
or
c) n=1, X represents an oxygen atom and Y represents a C₁ to C₄ linear or branched alkyl radical, or X represents a NR⁵ group, R⁵ standing for a lower alkyl radical, and Y stands for a linear or branched C₁ to C₄ alkyl radical ;
and wherein
R² represents hydrogen or a saturated or unsaturated, linear or branched C₁ to C₈ radical derived from a hydrocarbon ;
R³ and R⁴ are taken separately and each represents hydrogen or a saturated or unsaturated, linear or branched C₁ to C₈ radical derived from a hydrocarbon, or are taken together to form a five-membered or six-membered ring which also contains the carbon atoms of the ethylenic bond.

13. Catalyst according to claim 12, wherein, if applicable, the substrate is a compound of formula 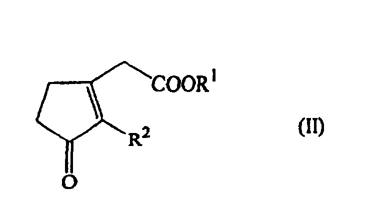 in which R¹ is a linear of branched alkyl radical from C₁ to C₄ and R² is a saturated or unsaturated, linear or branched, C₁ to C₈ hydrocarbon rest.

14. Catalyst according to claim 12, wherein, if applicable, the substrate is a compound of formula 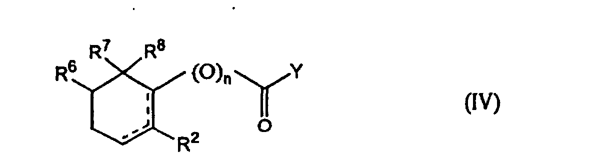 having a double bond in one of the positions indicated by the dotted lines and wherein :
a) n=0 and Y represents a OR¹ group, R¹ standing for a C₁ to C₄ linear or branched alkyl radical ;
or
b) n=1, and Y represents a C₁ to C₄ linear or branched alkyl radical ;
and wherein R² is a saturated or unsaturated, linear of branched, C₁ to C₈ hydrocarbon rest and R⁶, R⁷ and R⁸ represent each hydrogen or a lower alkyl radical.

15. Catalyst according to claim 13, wherein the substrate is methyl 3-oxo-2-pentyl-1-cyclopentene-1-acetate.

16. Catalyst according to claim 14, wherein the substrate is a compound of formula (IV) in which n=0 and Y represents a OR¹ group, R¹ representing a methyl or ethyl group, R² is methyl and R⁶, R⁷, R⁸ are identical or different and represent each hydrogen or a methyl group.

17. Catalyst according to any one of claims 12 to 16, wherein the solvent comprises dichloromethane and a small amount of substrate.

18. Catalyst according to claim 11 or 12, wherein the non-coordinating or weakly coordinating medium consists of methyl acetate.

19. Catalyst according to claim 13, wherein the Ru(II) complex is [(COD)Ru(2-methallyl)₂] or [Ru(COD)(COT)], the chelating diphosphine is (R,R)-(-)-Me-DuPHOS, the acid is HBF₄.etherate and the non-coordinating or weakly coordinating medium comprises dichloromethane and/or methyl 3-oxo-2-pentyl-1-cyclopentene-1-acetate.

20. Catalyst according to claim 13, wherein the Ru(II) complex is [Ru(COD)(COT)], the chelating diphosphine is (R)-(S)-JOSIPHOS, the acid is HBF₄.etherate and the non-coordinating or weakly coordinating medium comprises methyl tert-butyl ether and/or methyl 3-oxo-2-pentyl-1-cyclopentene-1-acetate.

21. A catalyst according to claim 1 or 2, wherein the oxygen content of the reaction atmosphere is not above 5 to 10 ppm.

22. A ruthenium catalyst of formula
[Ru(P*-P*)(H) (triene)]⁺ X⁻ (V)
wherein P*-P* represents a chelating chiral diphosphine, X represents a non-coordinating anion and "triene" stands for cyclohepta-1,3,5-triene or cycloocta-1,3,5-triene.

23. Catalyst according to claim 22, wherein the bis(phosphine) ligand is (R,R)-(-)-Me-DuPHOS or (R)-(S)-JOSIPHOS.

24. Catalyst according to claim 22 or 23, wherein the anion is BF₄⁻, PF₆⁻, SbF₆⁻ or AsF₆⁻, and preferably BF₄⁻.

25. Process for the preparation of a ruthenium catalyst, in which an appropriate Ru(II) complex and a chelating diphosphine, present in equimolar amounts, and an acid comprising a non-coordinating anion are reacted under an atmosphere having an oxygen content lower than 200 ppm, and in a non-coordinating or weakly coordinating medium, wherein the non-coordinating anion is used in an amount comprised between 0.95 and 1.10 molar equivalent per mole of the Ru(II) complex.

26. Process according to claim 25, wherein the non-coordinating or weakly coordinating medium comprises a substrate of formula (II) as defined in claim 13 or of formula (IV) as defined in claim 14.

27. Use of a ruthenium catalyst according to any one of claims 1 to 24, for the hydrogenation of carbon-carbon double bonds.

28. Use according to claim 27, wherein a substrate of formula (III) as defined in claim 12 is hydrogenated.

29. Process for the preparation of a compound of formula 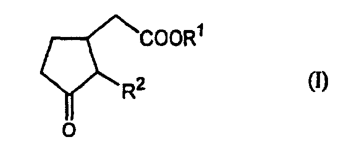 wherein R¹ is a linear of branched C₁ to C₄ alkyl radical and R² is a saturated or unsaturated, linear or branched C₁ to C₈ hydrocarbon rest, essentially in the form of an isomer of cis-configuration, **characterized in that** a substrate of formula 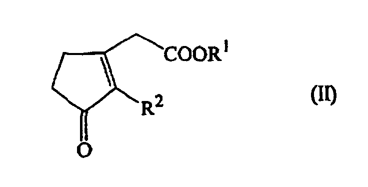 in which R¹ and R² have the meaning indicated above, is hydrogenated in the presence of a Ru catalyst according to any one of claims 1 to 24 and at a hydrogen pressure comprised between atmospheric pressure and 500 bar.

30. A process for the preparation of a compound of formula 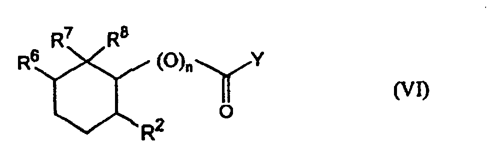 wherein the dotted lines and the symbols n, Y, R², R⁶, R⁷ and R⁸ are defined as in formula (IV), essentially in the form of an isomer of cis-configuration, **characterized in that** a substrate of formula (IV), as defined in claim 14, is hydrogenated in the presence of a ruthenium catalyst according to any one of claims 1 to 24 and at a hydrogen pressure comprised between atmospheric pressure and 500 bar.

31. A process according to claim 29, respectively 30, wherein there is used a catalyst as in claim 1 or 2, to provide the compound of formula (I), respectively (VI), essentially in the form of a stereoisomer of cis-configuration.

32. A process according to claim 29, respectively 30, wherein there is used a catalyst as in claim 3, to provide the compound of formula (I), respectively (VI), essentially in the form of an optically active isomer.

33. A process according to any one of claims 29 to 32, wherein the hydrogenation is carried out in a non-coordinating or weakly coordinating solvent under the reaction conditions.

34. A process according to any one of claims 29 to 33, wherein the ruthenium catalyst is formed in situ, optionally in the presence of the substrate.

35. A process according to claim 33 or 34, wherein the hydrogenation is carried out in a solvent selected from the group consisting of dichloromethane, dichloroethane, ethyl pivalate, methyl acetate, ethyl acetate, isopropyl acetate, acetone, 2-butanone, 3-pentanone, hexane, heptane, cyclohexane, cycloheptane and methyl tert-butyl ether and mixtures thereof.

36. A process according to claim 35, wherein the solvent is, or comprises, dichloromethane and there is used a catalyst as defined in claim 19.

37. A process according to claim 35, wherein there is used a catalyst as defined in claim 20 and the solvent is or comprises heptane, cycloheptane or methyl tert-butyl ether.

38. A process according to any one of claims 29 to 35, wherein the hydrogenation is carried out in a medium comprising a catalyst according to any one of claims 22 to 24.

39. A process according to any one of claims 29 to 38, wherein the catalyst is used in a concentration of 0.01 to 1 molar %, preferably 0.01 to 0.5 molar %, more preferably 0.02 to 0.1 molar %, relative to the substrate.

40. A process according to any one of claims 29 to 32, wherein the substrate subjected to hydrogenation is methyl 3-oxo-2-pentyl-1-cyclopentene-1-acetate.

41. A process according to claim 40, wherein the hydrogenation is carried out in a medium comprising a catalyst according to any one of claims 22 to 24.

42. A process according to claim 25, wherein the reaction atmosphere has an oxygen content not above 5 to 10 ppm.

## Revendications

1. Un catalyseur de ruthénium comprenant le produit de réaction d'un complexe de Ru(II) approprié, une diphosphine chélatante et un acide comprenant un anion non-coordinant, lesdits complexe de Ru(II) et diphosphine chélatante étant utilisés en quantités équimolaires, la réaction étant effectuée dans un milieu non-coordinant ou faiblement coordinant et sous une atmosphère ayant une teneur en oxygène inférieure à 200 ppm, et l'acide comprenant un anion non-coordinant étant utilisé en quantités comprises entre 0.95 et 1.10 équivalent molaire par mole de complexe de Ru(II).

2. Un catalyseur selon la revendication 1, consistant essentiellement en ce produit de réaction.

3. Un catalyseur selon la revendication 1 ou 2, **caractérisé en ce que** la diphosphine chélatante est un ligand diphosphine chiral.

4. Un catalyseur selon la revendication 3, **caractérisé en ce que** le complexe de Ru(II) est sélectionné dans le groupe des composés de Ru(II) du type [(diene)Ru(allyl)₂] ou [bis(pentadienyl)Ru].

5. Un catalyseur selon la revendication 3, **caractérisé en ce que** le complexe de ruthénium est [(COD)Ru(2-methallyl)₂] ou [Ru(COD)(COT)].

6. Un catalyseur selon l'une des revendications 2 à 5, **caractérisé en ce que** la diphosphine chélatante est sélectionnée dans le groupe consistant en les ligands chiraux connus sous les abréviations Me-DuPHOS, Et-DuPHOS, BINAP, TolBINAP, SKEWPHOS et JOSIPHOS.

7. Un catalyseur selon la revendication 6, **caractérisé en ce que** le ligand phosphine bidenté est sélectionné dans le groupe des diphosphines chirales connues sous les abréviations Me-DuPHOS, SKEWPHOS et JOSIPHOS.

8. Un catalyseur selon la revendication 7, **caractérisé en ce que** le ligand est (R,R)-(-)-Me-DuPHOS, (R)-(S)-JOSIPHOS ou (R)-(S)-CF₃-JOSIPHOS, et de préférence (R,R)-(-)-MeDuPHOS ou (R)-(S)-JOSIPHOS.

9. Un catalyseur selon l'une des revendications précédentes, **caractérisé en ce que** l'anion non-coordinant est dérivé d'un acide de formule HX, dans lequel X est sélectionné dans le groupe constitué par BF₄⁻, B[3,5-(CF₃)₂C₆H₄]₄⁻, PF₆⁻, SbF₆⁻ et AsF₆⁻.

10. Un catalyseur selon la revendication 9, **caractérisé en ce que** l'anion non-coordinant est BF₄⁻, préférablement dérivé du HBF₄⁻ éthérate.

11. Un catalyseur selon l'une des revendications 1 à 10, **caractérisé en ce que** le milieu faiblement coordinant est un solvant sélectionné dans le groupe constitué par les dichlorométhane, dichloroéthane, pivalate d'éthyle, acétate de méthyle, acétate d'éthyle, acétate d'isopropyle, acétone, 2-butanone, 3-pentanone, hexane, heptane, cyclohexane, cycloheptane et tert-butyle méthyle éther et leurs mélanges.

12. Un catalyseur selon l'une des revendications 1 à 11, **caractérisé en ce que** le milieu non-coordinant ou faiblement coordinant est constitué d'un solvant organique non-coordinant ou faiblement coordinant et/ou d'un substrat de formule 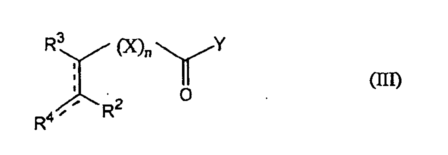 ayant une double liaison dans l'une des positions indiquées par la ligne pointillée et dans laquelle
a) n=0 et Y représente un hydrogène, un radical alkyle de C₁ à C₄ linéaire ou branché ou un groupe OR¹, R¹ représentant un radical alkyle inférieur linéaire ou branché;
ou
b) n=1, X représente un groupe CH₂ et Y représente un group OR¹ dans lequel R¹ possède la signification indiquée sous a);
ou
c) n=1, X représente un atome d'oxygène et Y représente un radical alkyle de C₁ à C₄ linéaire ou branché, ou X représente un groupe NR⁵, R⁵ représentant un radical alkyle inférieur, et Y représente un radical alkyle de C₁ à C₄ linéaire ou branché;
et dans laquelle
R² représente un hydrogène ou un radical de C₁ à C₈ saturé ou insaturé, linéaire ou branché, dérivé d'un hydrocarbure;
R³ et R⁴, si pris séparément, représentent chacun un hydrogène, un radical de C₁ à C₈ saturé ou insaturé, linéaire ou branché, dérivé d'un hydrocarbure ou, si pris ensemble, forment un cycle de cinq ou six atomes contenant aussi les atomes de carbone de la double liaison.

13. Un catalyseur selon la revendication 12, **caractérisé en ce que**, si applicable, le substrat est un composé de formule 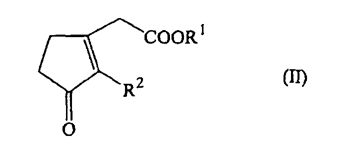 dans laquelle R¹ est un radical alkyle de C₁ à C₄, linéaire ou branché, et R² est un résidu d'hydrocarbure saturé ou insaturé, linéaire ou branché, de C₁ à C₈.

14. Un catalyseur selon la revendication 12, **caractérisé en ce que**, si applicable, le substrat est un composé de formule 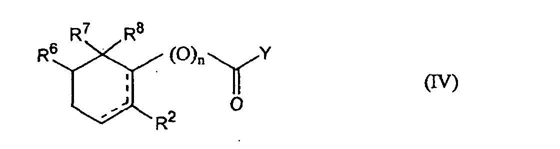 ayant une double liaison dans l'une des positions indiquées par la ligne pointillée et dans laquelle
a) n=0 et Y représente un groupe OR¹, R¹ représentant un radical alkyle de C₁ à C₄ linéaire ou branché;
ou
b) n=1, et Y représente un radical alkyle de C₁ à C₄ linéaire ou branché;
et dans laquelle R² est un résidu d'hydrocarbure saturé ou insaturé, linéaire ou branché, de C₁ à C₈ et R⁶, R⁷ et R⁸ représentent chacun un hydrogène ou un radical alkyle inférieur.

15. Un catalyseur selon la revendication 13, **caractérisé en ce que** le substrat est le 3-oxo-2-péntyl-1-cyclopentène-1-acétate de méthyle.

16. Un catalyseur selon la revendication 14, **caractérisé en ce que** le substrat est un composé de formule (IV) dans laquelle n=0 et Y représente un groupe OR¹, R¹ représentant un groupe méthyle ou éthyle, R² est un méthyle et R⁶, R⁷, R⁸ sont identiques ou différents et représentent chacun un hydrogène ou un groupe méthyle.

17. Un catalyseur selon l'une des revendications 12 à 16, **caractérisé en ce que** le solvant comprend du dichlorométhane et une faible quantité de substrat.

18. Un catalyseur selon la revendication 11 ou 12, **caractérisé en ce que** le milieu non-coordinant ou faiblement coordinant consiste en l'acétate de méthyle.

19. Un catalyseur selon la revendication 13, **caractérisé en ce que** le complexe de Ru(II) est [(COD)Ru(2-methallyl)₂) ou [Ru(COD)(COT)], la diphosphine chélatante est (R,R)-(-)-Me-DuPHOS, l'acide est HBF₄⁻ éthérate et le milieu non-coordinant ou faiblement coordinant comprend du dichlorométhane et/ou 3-oxo-2-pentyl-1-cyclopentene-1-acétate de méthyle.

20. Un catalyseur selon la revendication 13, caractérisé en ce le complexe de Ru(II) est [Ru(COD)(COT)], la diphosphine chélatante est (R)-(S)-JOSIPHOS, l'acide est HBF₄⁻ éthérate et le milieu non-coordinant ou faiblement coordinant comprend le méthyle tert-butyle éther et/ou le 3-oxo-2-pentyl-1-cyclopentene-1-acétate de méthyle.

21. Un catalyseur selon la revendication 1 ou 2, **caractérisé en ce que** la teneur en oxygène de l'atmosphère de la réaction n'est pas supérieure à 5 à 10 ppm.

22. Un catalyseur de ruthénium de formule
[Ru(P*-P*) (H) (triène)]⁺ X- (V)
dans lequel P*-P* représente une diphosphine chélatante chirale, X représente un anion non-coordinant et "triène" représente le cyclohepta-1,3,5-triène ou cycloocta-1,3,5-triène.

23. Un catalyseur selon la revendication 22, **caractérisé en ce que** le ligand bis(phosphine) est (R,R)-(-)-Me-DuPHOS ou (R)-(S)-JOSIPHOS.

24. Un catalyseur selon la revendication 22 ou 23, **caractérisé en ce que** l'anion est BF₄⁻, PF₆⁻, SbF₆⁻ ou AsF₆⁻, préférablement BF₄⁻.

25. Un procédé pour la préparation d'un catalyseur de ruthénium, dans lequel un complexe approprié de Ru(II) et une diphosphine chélatante, présents en quantités équimolaires, et un acide comprenant un anion non-coordinant, sont réagis sous une atmosphère ayant une teneur en oxygène inférieure à 200 ppm, et dans un milieu non-coordinant ou faiblement coordinant, et dans lequel l'anion non-coordinant est utilisé en quantités comprises entre 0.95 et 1.10 équivalent molaire par mole de complexe de Ru(II).

26. Un procédé selon la revendication 25, **caractérisé en ce que** le milieu non-coordinant ou faiblement coordinant comprend un substrat de formule (II) telle que définie à la revendication 13, ou de formule (IV) telle que définie à la revendication 14.

27. Utilisation d'un catalyseur de ruthénium selon l'une des revendications 1 à 24 pour l'hydrogénation de doubles liaisons carbone-carbone.

28. Utilisation selon la revendication 27, **caractérisé en ce qu'**un substrat de formule (III) telle que définie à la revendication 12 est hydrogéné.

29. Un procédé pour la préparation d'un composé de formule 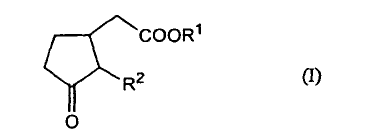 dans laquelle R¹ est un radical alkyle de C₁ à C₄, linéaire ou branché, et R² est un résidu d'hydrocarbure saturé ou insaturé, linéaire ou branché, de C₁ à C₈, essentiellement sous la forme d'un isomère de configuration cis, **caractérisé en ce que** le substrat de formule 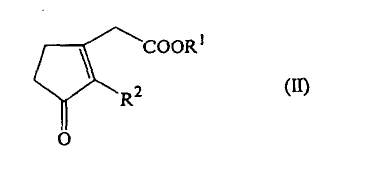 dans laquelle R¹ et R² possèdent la signification indiquée ci-dessus, est hydrogéné en présence d'un catalyseur de Ru selon l'une des revendications 1 à 24 et à une pression d'hydrogène comprise entre la pression atmosphérique et 500 bar.

30. Un procédé pour la préparation d'un composé de formule 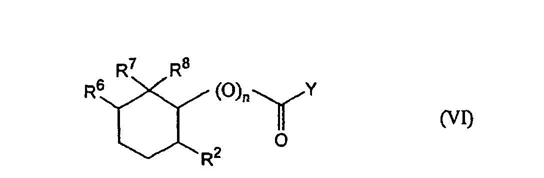 dans laquelle les lignes pointillés et les symboles n, Y, R², R⁶, R⁷ et R⁸ sont tels que définis à la formule (IV), essentiellement sous la forme d'un isomère de configuration cis, **caractérisé en ce qu'**un substrat de formule (IV), tel que défini à la revendication 14, est hydrogéné en présence d'un catalyseur de ruthénium selon l'une des revendications 1 à 24 et sous une pression d'hydrogène comprise entre la pression atmosphérique et 500 bars.

31. Un procédé selon la revendication 29, respectivement 30, **caractérisé en ce qu'**on utilise un catalyseur tel que défini à la revendication 1 ou 2, pour former le produit de formule (I), respectivement (VI), essentiellement sous la forme d'un stéréoisomère de configuration cis.

32. Un procédé selon la revendication 29, respectivement 30, **caractérisé en ce qu'**on utilise un catalyseur tel que défini à la revendication 3, pour former le produit de formule (I), respectivement (VI), essentiellement sous la forme d'un isomère optiquement actif.

33. Un procédé selon l'une des revendications 29 à 32, **caractérisé en ce que** l'hydrogénation est effectuée dans une solvant qui est non-coordinant ou faiblement coordinant sous les conditions de réaction.

34. Un procédé selon l'une des revendications 29 à 33, **caractérisé en ce que** le catalyseur de ruthénium est formé in situ, éventuellement en présence d'un substrat.

35. Un procédé selon la revendication 33 ou 34, **caractérisé en ce que** l'hydrogénation est effectuée dans une solvant sélectionné dans le groupe constitué par les dichlorométhane, dichloroéthane, pivalate d'éthyle, acétate de méthyle, acétate d'éthyle, acétate d'isopropyle, acétone, 2-butanone, 3-péntanone, hexane, heptane, cyclohexane, cycloheptane et tert-butyle méthyle éther et leurs mélanges.

36. Un procédé selon la revendication 35, **caractérisé en ce que** le solvant est, ou comprend, du dichlorométhane et **en ce qu'**on utilise un catalyseur tel que défini à la revendication 19.

37. Un procédé selon la revendication 35, **caractérisé en ce qu'**on utilise un catalyseur tel que défini à la revendication 20 et le solvant est, ou comprend, l'heptane, le cycloheptane ou le méthyle tert-butyle éther.

38. Un procédé selon l'une des revendications 29 à 35, **caractérisé en ce que** l'hydrogénation est effectuée dans un milieu comprenant un catalyseur selon l'une des revendications 22 à 24.

39. Un procédé selon l'une des revendications 29 à 38, **caractérisé en ce que** le catalyseur est utilisé en une concentration comprise entre 0.01 et 1% molaire, de préférence 0.01 et 0.5% molaire, et plus préférablement entre 0.02 et 0.1 % molaire, par rapport au substrat.

40. Un procédé selon l'une des revendications 29 à 32, **caractérisé en ce que** le substrat utilisé dans l'hydrogénation est le 3-oxo-2-péntyl-1-cyclopentène-1-acétate de méthyle.

41. Un procédé selon la revendication 40, **caractérisé en ce que** l'hydrogénation est effectuée dans un milieu comprenant un catalyseur selon l'une des revendications 22 à 24.

42. Un procédé selon la revendication 25, **caractérisé en ce que** l'atmosphère possède une teneur en oxygène qui ne dépasse pas 5 à 10 ppm.

## Patentansprüche

1. Rutheniumkatalysator, umfassend das Reaktionsprodukt aus einem geeigneten Ruthenium(II)-Komplex, einem chelatbildenden Diphosphin und einer Säure, die ein nicht-koordinierendes Anion umfaßt, wobei der Ru(II)-Komplex und das chelatbildende Diphosphin in äquimolaren Mengen vorliegen, die Reaktion in einem nicht-koordinierenden oder schwach koordienierenden Medium und unter einer Atmosphäre mit einem Sauerstoffgehalt von unter 200 ppm stattfindet, und die Säure, die ein nicht-koordinierendes Anion umfasst, in einer Menge verwendet wird, die zwischen 0,95 und 1,10 Moläquivalenten pro Mol des Ru(II)-Komplexes liegt.

2. Katalysator nach Anspruch 1, der im wesentlichen aus diesem Reaktionsprodukt besteht.

3. Katalysator nach Anspruch 1 oder 2, wobei das chelatbildende Diphosphin ein chiraler Diphosphin-Ligand ist.

4. Katalysator nach Anspruch 3, wobei der Ru(II)-Komplex ausgewählt ist aus der Gruppe aus Ru(II)-Verbindungen des Typs [(Dien)Ru(allyl)₂] oder [Bis(pentadienyl)Ru].

5. Katalysator nach Anspruch 3, wobei der Rutheniumkomplex [(COD)Ru(2-methallyl)₂] oder [Ru(COD)(COT)] ist.

6. Katalysator nach einem der Ansprüche 2 bis 5, wobei das chelatbildende Diphosphin ausgewählt ist aus der Gruppe bestehend aus den chiralen Liganden, die unter den Abkürzungen Me-DuPHOS, Et-DuPHOS, BINAP, TolBINAP, SKEWPHOS und JOSIPHOS bekannt sind.

7. Katalysator nach Anspruch 6, wobei der zweizähnige Phosphinligand ausgewählt ist aus der Gruppe chiraler Diphosphine, die unter den Abkürzungen Me-DuPHOS, SKEWPHOS und JOSIPHOS bekannt sind.

8. Katalysator nach Anspruch 7, wobei der Ligand (R,R)-(-)-Me-DuPHOS, (R)-(S)-JOSIPHOS oder (R)-(S)-CF₃-JOSIPHOS und vorzugsweise (R,R)-(-)-MeDuPHOS oder (R)-(S)-JOSIPHOS ist.

9. Katalysator nach einem der vorangehenden Ansprüche, wobei das nicht-koordinierende Anion aus einer Säure der Formel HX stammt, wobei X ausgewählt ist aus der Gruppe bestehend aus BF₄⁻, B[3,5-(CF₃)₂C₆H₄]₄⁻, PF₆⁻, SbF₆⁻ und AsF₆⁻.

10. Katalysator nach Anspruch 9, wobei das nicht-koordinierende Anion BF₄⁻ ist, das vorzugsweise aus HBF₄-Etherat stammt.

11. Katalysator nach einem der Ansprüche 1 bis 10, wobei das schwach koordinierende Medium ein Lösungsmittel ist, das ausgewählt ist aus der Gruppe bestehend aus Dichlormethan, Dichlorethan, Ethylpivalat, Methylacetat, Ethylacetat, Isopropylacetat, Aceton, 2-Butanon, 3-Pentanon, Hexan, Heptan, Cyclohexan, Cycloheptan und Methyl-tert.-butylether und Mischungen davon.

12. Katalysator nach einem der Ansprüche 1 bis 11, wobei das nicht-koordinierende oder schwach koordinierende Medium aus einem nicht-koordinierenden oder schwach koordinierenden organischen Lösungsmittel und/oder einem Substrat der Formel 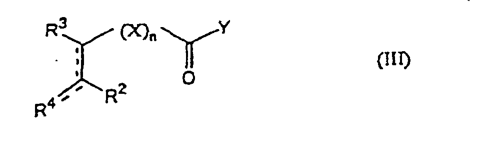 mit einer Doppelbindung in einer der durch die gestrichelten Linien angegebenen Positionen gebildet ist, wobei
a) n=0 und Y Wasserstoff, ein lineares oder verzweigtes C₁-C₄-Alkylradikal oder eine OR¹-Gruppe darstellt, worin R¹ für ein lineares oder verzweigtes Niederalkylradikal steht; oder
b) n=1, X eine CH₂-Gruppe darstellt und Y für eine OR¹-Gruppe steht, wobei R¹ die in a) angegebene Bedeutung hat;
oder
c) n=1, X ein Sauerstoffatom darstellt und Y ein lineares oder verzweigtes C₁-C₄-Alkylradikal darstellt, oder X eine NR⁵-Gruppe darstellt, worin R⁵ für ein Niederalkylradikal steht, und Y für ein lineares oder verzweigtes C₁-C₄-Alkylradikal steht;
und wobei
R² Wasserstoff oder ein von einem Kohlenwasserstoff abgeleitetes gesättigtes oder ungesättigtes lineares oder verzweigtes C₁-C₈-Radikal darstellt;
R³ und R⁴ jeweils für sich Wasserstoff oder ein von einem Kohlenwasserstoff abgeleitetes gesättigtes oder ungesättigtes lineares oder verzweigtes C₁-C₈-Radikal darstellen oder zusammen einen fünfgliedrigen oder sechsgliedrigen Ring bilden, welcher auch die Kohlenstoffatome der Ethylenbindung enthält.

13. Katalysator nach Anspruch 12, wobei das Substrat, falls zutreffend, eine Verbindung der Formel 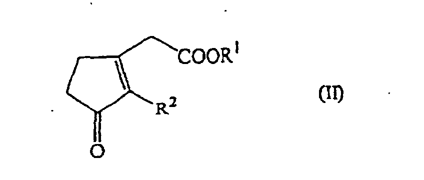 ist, wobei R¹ ein lineares oder verzweigtes C₁-C₄-Alkylradikal von ist und R² ein gesättigter oder ungesättigter linearer oder verzweigter C₁-C₈-Kohlenwasserstoffrest ist.

14. Katalysator nach Anspruch 12, wobei das Substrat, falls zutreffend, eine Verbindung der Formel 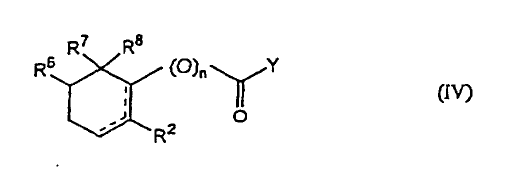 mit einer Doppelbindung in einer der durch die gestrichelten Linien angegebenen Positionen ist, wobei
a) n=0 und Y eine OR¹-Gruppe darstellt, worin R¹ für ein lineares oder verzweigtes C₁-C₄-Alkylradikal steht;
oder
b) n=1 und Y ein lineares oder verzweigtes C₁-C₄-Alkylradikal darstellt; und wobei R² ein gesättigter oder ungesättigter linearer oder verzweigter C₁-C₈-Kohlenwasserstoffrest ist und R⁶, R⁷ und R⁸ jeweils Wasserstoff oder ein Niederalkylradikal darstellen.

15. Katalysator nach Anspruch 13, wobei das Substrat Methyl-3-oxo-2-pentyl-1-cyclopenten-1-acetat ist.

16. Katalysator nach Anspruch 14, wobei das Substrat eine Verbindung der Formel (IV) ist, wobei n=0 und Y eine OR¹-Gruppe darstellt, worin R¹ eine Methyl- oder Ethylgruppe darstellt, R² Methyl ist und R⁶, R⁷, R⁸ gleich oder verschieden sind und jeweils Wasserstoff oder eine Methylgruppe darstellen.

17. Katalysator nach einem der Ansprüche 12 bis 16, wobei das Lösungsmittel Dichlormethan und eine kleine Menge Substrat umfaßt.

18. Katalysator nach Anspruch 11 oder 12, wobei das nicht-koordinierende oder schwach koordinierende Medium aus Methylacetat besteht.

19. Katalysator nach Anspruch 13, wobei der Ru(II)-Komplex [(COD)Ru(2-methallyl)₂] oder [Ru(COD)(COT)] ist, das chelatbildende Diphosphin (R,R)-(-)-Me-DuPHOS ist, die Säure HBF₄•Etherat ist und das nicht-koordinierende oder schwach koordinierende Medium Dichlormethan und/oder Methyl-3-oxo-2-pentyl-1-cyclopenten-1-acetat umfaßt.

20. Katalysator nach Anspruch 13, wobei der Ru(II)-Komplex [Ru(COD)(COT)] ist, das chelatbildende Diphosphin (R)-(S)-JOSIPHOS ist, die Säure HBF₄•Etherat ist und das nicht-koordinierende oder schwach koordinierende Medium Methyl-tert.-butylether und/oder Methyl-3-oxo-2-pentyl-1-cyclopenten-1-acetat umfaßt.

21. Katalysator nach Anspruch 1 oder 2, wobei der Sauerstoffgehalt der Reaktionsatmosphäre nicht mehr als 5 bis 10 ppm beträgt.

22. Rutheniumkatalysator der Formel
[Ru(P*-P*)(H)(trien)]⁺X⁻ (V)
wobei P*-P* ein chelatbildendes chirales Diphosphin darstellt, X ein nicht-koordinierendes Anion darstellt und "Trien" für Cyclohepta-1,3,5-trien oder Cycloocta-1,3,5-trien steht.

23. Katalysator nach Anspruch 22, wobei der Bis(phosphin)-Ligand (R,R)-(-)-Me-DuPHOS oder (R)-(S)-JOSIPHOS ist.

24. Katalysator nach Anspruch 22 oder 23, wobei das Anion BF₄⁻, PF₆⁻, SbF₆⁻ oder AsF₆⁻ und vorzugsweise BF₄⁻ ist.

25. Verfahren zur Herstellung eines Rutheniumkatalysators, bei welchem ein geeigneter Ru(II)-Komplex und ein chelatbildendes Diphosphin, die in äquimolaren Mengen vorliegen, und eine Säure, die ein nicht-koordinierendes Anion umfaßt, unter einer Atmosphäre mit einem Sauerstoffgehalt von unter 200 ppm und in einem nicht-koordinierenden oder schwach koordinierenden Medium umgesetzt werden, wobei das nicht-koordinierende Anion in einer Menge verwendet wird, die zwischen 0,95 und 1,10 Moläquivalenten pro Mol des Ru(II)-Komplexes liegt.

26. Verfahren nach Anspruch 25, wobei das nicht-koordinierende oder schwach koordinierende Medium ein Substrat mit der wie in Anspruch 13 definierten Formel (II) oder der wie in Anspruch 14 definierten Formel (IV) umfaßt.

27. Verwendung eines Rutheniumkatalysators nach einem der Ansprüche 1 bis 24 zur Hydrierung von Kohlenstoff-Kohlenstoff-Doppelbindungen.

28. Verwendung nach Anspruch 27, wobei das wie in Anspruch 12 definierte Substrat der Formel (III) hydriert wird.

29. Verfahren zur Herstellung einer Verbindung der Formel 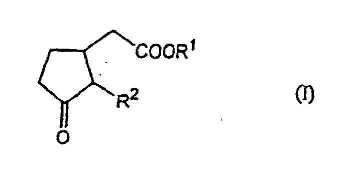 wobei R¹ ein lineares oder verzweigtes C₁-C₄-Alkylradikal ist und R₂ ein gesättigter oder ungesättigter linearer oder verzweigter C₁-C₈-Kohlenwasserstoffrest ist, im wesentlichen in Form eines Isomers der cis-Konfiguration, **dadurch gekennzeichnet, daß** ein Substrat der Formel 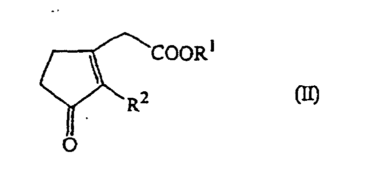 worin R₁ und R₂ die oben angegebene Bedeutung haben, in Gegenwart eines Ru-Katalysators nach einem der Ansprüche 1 bis 24 und bei einem Wasserstoffdruck, der zwischen Atmosphärendruck und 500 bar liegt, hydriert wird.

30. Verfahren zur Herstellung einer Verbindung der Formel 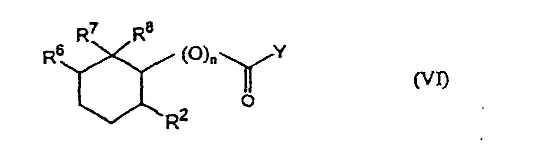 wobei die gestrichelten Linien und die Symbole n, Y, R², R⁶, R⁷ und R⁸ wie in Formel (IV) definiert sind, im wesentlichen in Form eines Isomers der cis-Konfiguration, **dadurch gekennzeichnet, daß** ein Substrat der Formel (IV), wie in Anspruch 14 definiert, in Gegenwart eines Rutheniumkatalysators nach einem der Ansprüche 1 bis 24 und bei einem Wasserstoffdruck, der zwischen Atmosphärendruck und 500 bar liegt, hydriert wird.

31. Verfahren nach Anspruch 29 bzw. 30, wobei ein Katalysator wie in Anspruch 1 oder 2 verwendet wird, zur Bereitstellung der Verbindung der Formel (I) bzw. (VI) im wesentlichen in Form eines Stereoisomers der cis-Konfiguration.

32. Verfahren nach Anspruch 29 bzw. 30, wobei ein Katalysator wie in Anspruch 3 verwendet wird, zur Bereitstellung der Verbindung der Formel (I) bzw. (VI) im wesentlichen in Form eines optisch aktiven Isomers.

33. Verfahren nach einem der Ansprüche 29 bis 32, wobei die Hydrierung in einem nicht-koordinierenden oder schwach koordinierenden Lösungsmittel unter den Reaktionsbedingungen durchgeführt wird.

34. Verfahren nach einem der Ansprüche 29 bis 33, wobei der Rutheniumkatalysator in situ gebildet wird, gegebenenfalls in Gegenwart des Substrats.

35. Verfahren nach Anspruch 33 oder 34, wobei die Hydrierung in einem Lösungsmittel durchgeführt wird, das ausgewählt ist aus der Gruppe bestehend aus Dichlormethan, Dichlorethan, Ethylpivalat, Methylacetat, Ethylacetat, Isopropylacetat, Aceton, 2-Butanon, 3-Pentanon, Hexan, Heptan, Cyclohexan, Cycloheptan und Methyl-tert.-butylether und Mischungen davon.

36. Verfahren nach Anspruch 35, wobei das Lösungsmittel Dichlormethan ist oder umfaßt und ein wie in Anspruch 19 definierter Katalysator verwendet wird.

37. Verfahren nach Anspruch 35, wobei ein wie in Anspruch 20 definierter Katalysator verwendet wird und das Lösungsmittel Heptan, Cycloheptan oder Methyl-tert.butylether ist oder umfaßt.

38. Verfahren nach einem der Ansprüche 29 bis 35, wobei die Hydrierung in einem Medium durchgeführt wird, welches einen Katalysator nach einem der Ansprüche 22 bis 24 umfaßt.

39. Verfahren nach einem der Ansprüche 29 bis 38, wobei der Katalysator, bezogen auf das Substrat, in einer Konzentration von 0,01 bis 1 Mol-%, vorzugsweise 0,01 bis 0,5 Mol-%, besonders bevorzugt 0,02 bis 0,1 Mol-% verwendet wird.

40. Verfahren nach einem der Ansprüche 29 bis 32, wobei das Substrat, das der Hydrierung unterzogen wird, Methyl-3-oxo-2-pentyl-1-cyclopenten-1-acetat ist.

41. Verfahren nach Anspruch 40, wobei die Hydrierung in einem Medium durchgeführt wird, das einen Katalysator nach einem der Ansprüche 22 bis 24 umfaßt.

42. Verfahren nach Anspruch 25, wobei die Reaktionsatmosphäre einen Sauerstoffgehalt von nicht mehr als 5 bis 10 ppm aufweist.
